# EUROPEAN PATENT APPLICATION

(11) **EP 4 772 872 A1**
(43) Date of publication of application: **08.07.2026**
(21) Application number: 24859662.9
(22) Date of filing: 23.08.2024
(51) Int. Cl.: G01N 27/416, G01N 33/68

(54) **METHOD AND DEVICE FOR MEASURING BIOMOLECULE**

(30) Priority: 29.08.2023 US 202363579312 P
(71) Applicant: The University of Tokyo, Bunkyo-ku, Tokyo 113-8654 (JP); Provigate Inc., Tokyo 113-0023 (JP)
(72) Inventor: SAKATA, Toshiya, Tokyo 113-8654 (JP); TSENG, Alex Chi-Wei, Tokyo 113-8654 (JP); ITO, Narushi, Tokyo 113-0033 (JP)
(74) Representative: Michalski Hüttermann & Partner mbB
(86) International application number: PCT/JP2024/030105
(87) International publication number: WO 2025/047629

(57) **Abstract**

According to an embodiment of the present disclosure, a method for measuring a biomolecule is provided. The method comprises: providing a recognition unit comprising a conductive polymer and a molecular recognition pH indicator that acts as a dopant for the conductive polymer and recognizes a target biomolecule; introducing a sample containing the target biomolecule to the recognition unit; measuring an electrical property of the conductive polymer based on a reaction between the target biomolecule and the molecular recognition pH indicator that occurs when the target biomolecule is introduced; and determining an amount of the target biomolecule in the sample based on the measured electrical property.

## Description

### TECHNICAL FIELD

The present disclosure relates to a method and a device for measuring a biomolecule.

### BACKGROUND ART

In the field of biosensing, there is a demand for technology that measures biomolecules present in a solution, such as a body fluid, more accurately and more simply.

For example, a GA level has been attracting attention as an important index for blood glucose management. Technology for measuring the GA level simply and accurately is required. The GA level is a ratio of an amount of glycated albumin to a total amount of albumin. Biosensing technologies for glycated albumin have been developed. On the other hand, the total amount of albumin is generally measured using a BCP method, a BCG method, an HPLC method, an immunoturbidimetric method, an ELISA (Enzyme-Linked ImmunoSorbent Assay), a high-performance liquid chromatography (HPLC) precipitation method, or the like. However, all of these are liquid phase methods or in-solution measurement methods. Thus, the development of biosensors for measuring albumin has been delayed.

### SUMMARY

Herein, a need is recognized for a technology that simply and easily measures various substances (e.g., proteins, etc.) present in a body fluid using a biosensor, without being limited to a total amount of albumin.

In some embodiments of the present disclosure, a method for measuring a biomolecule is provided. In some embodiments, the method comprises providing a recognition unit comprising a conductive polymer and a molecular recognition pH indicator that acts as a dopant for the conductive polymer and recognizes a target biomolecule. In some embodiments, the method comprises introducing a sample containing the target biomolecule to the recognition unit. In some embodiments, the method comprises measuring an electrical property of the conductive polymer based on a reaction between the target biomolecule and the molecular recognition pH indicator that occurs when the target biomolecule is introduced. In some embodiments, the method comprises determining an amount of the target biomolecule in the sample based on the measured electrical property.

According to the above embodiments, for example, an amount of a biomolecule in a body fluid can be measured simply and accurately using a biosensor.

Further aspects and advantages of the present disclosure will become readily apparent to those skilled in the art from the following detailed description, wherein only exemplary embodiments of the present disclosure are shown and described. As will be realized, the present disclosure is capable of other and different embodiments, and its several details are capable of modifications in various obvious respects, all without departing from the present disclosure. Accordingly, the drawings and description are to be regarded as illustrative in nature, and not as restrictive.

### BRIEF DESCRIPTION OF DRAWINGS

FIG. 1 is a cyclic voltammogram of a PEDOT:BCP system for HSA solutions with multiple concentrations according to an example.
FIG. 2 is a graph showing a relationship between a concentration of the HSA solution and a height of an oxidation peak of the cyclic voltammogram shown in FIG. 1.
FIG. 3 is a schematic diagram showing a configuration and a function of a device according to an embodiment.
FIG. 4 is a schematic diagram showing a configuration and a function of a device according to an embodiment.
FIG. 5 is a schematic diagram showing a configuration and a function of a device according to an embodiment.
FIG. 6 is a schematic diagram showing a configuration of a device for measuring a GA level according to an embodiment.
FIG. 7 is a schematic diagram showing a configuration of a device for measuring a GA level according to an embodiment.
FIG. 8 is a schematic diagram showing a configuration of a device for measuring a GA level according to an embodiment.
FIG. 9A1 is a description regarding evaluation of a PEDOT:BCP electrochemical electrode in human serum albumin measurement by a dye-binding method (p1).

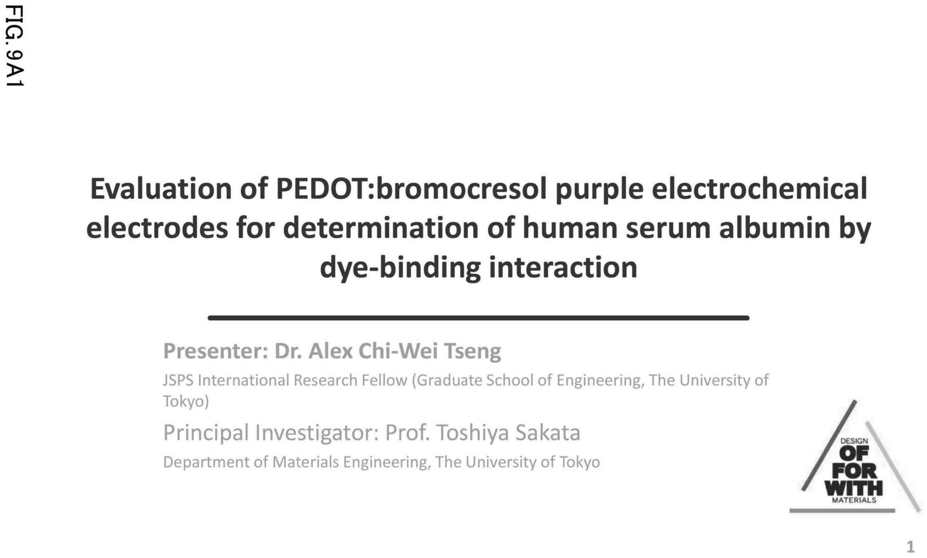

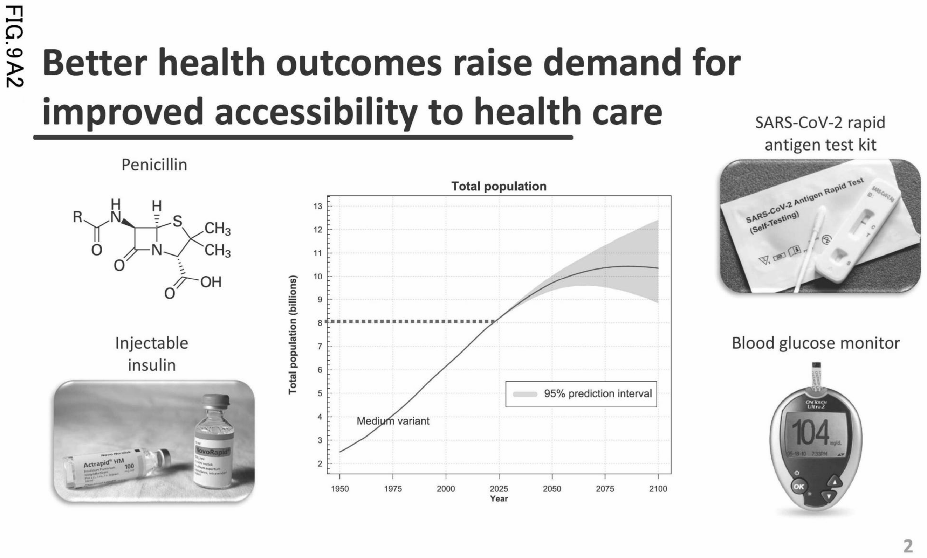

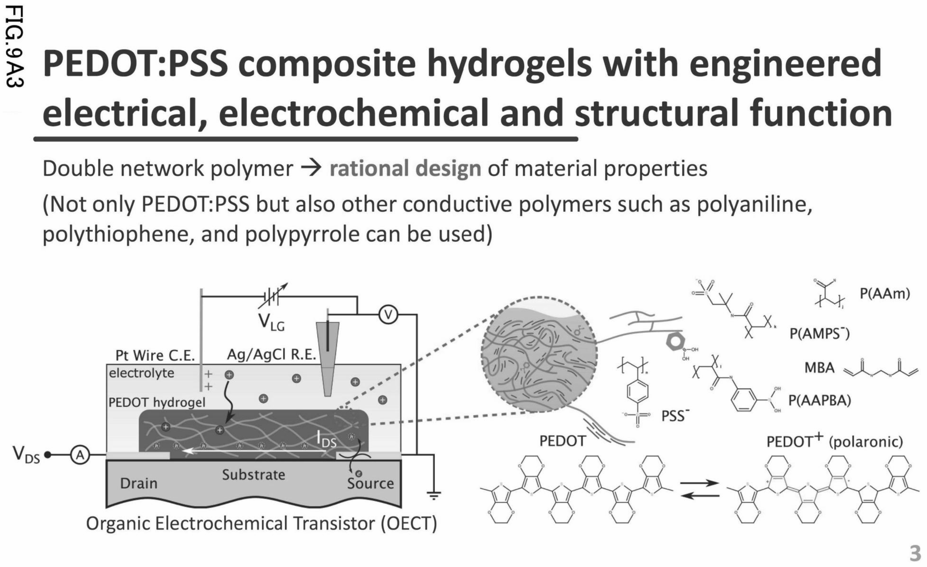

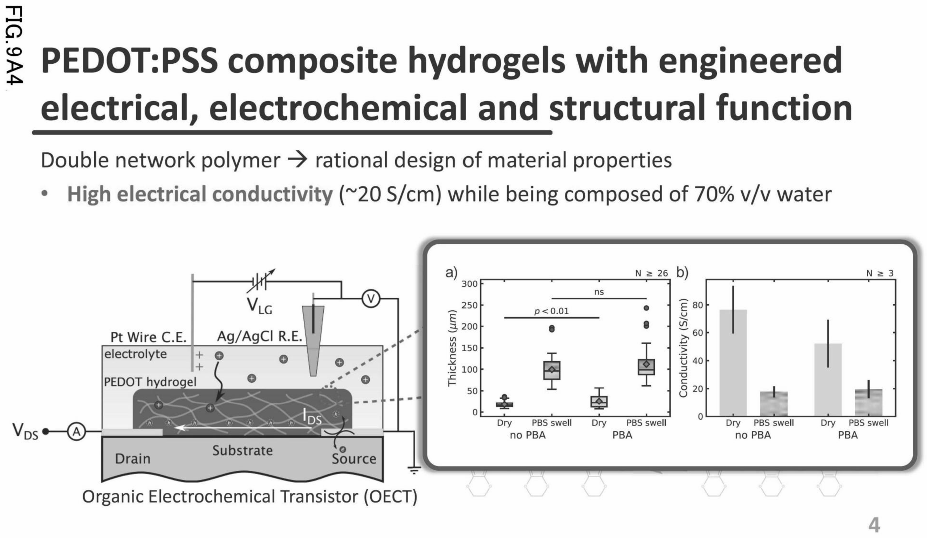

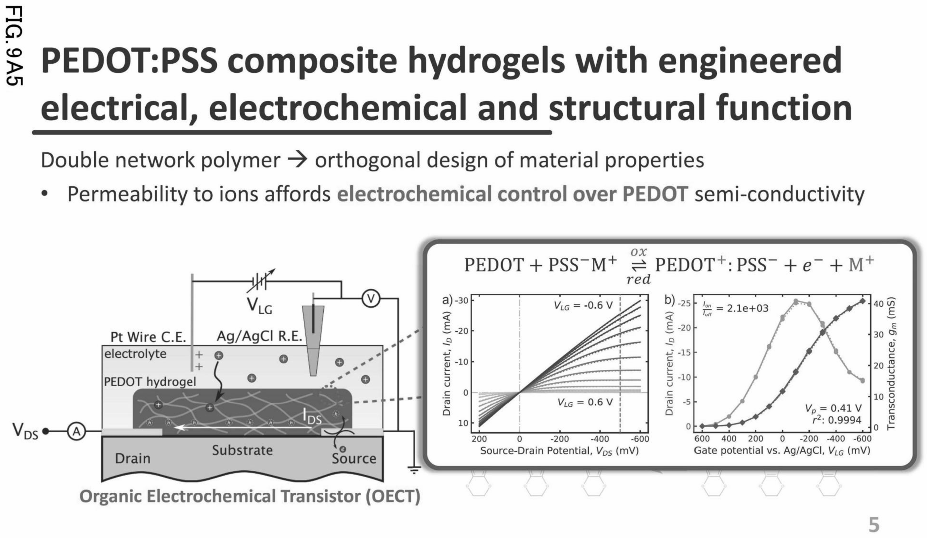

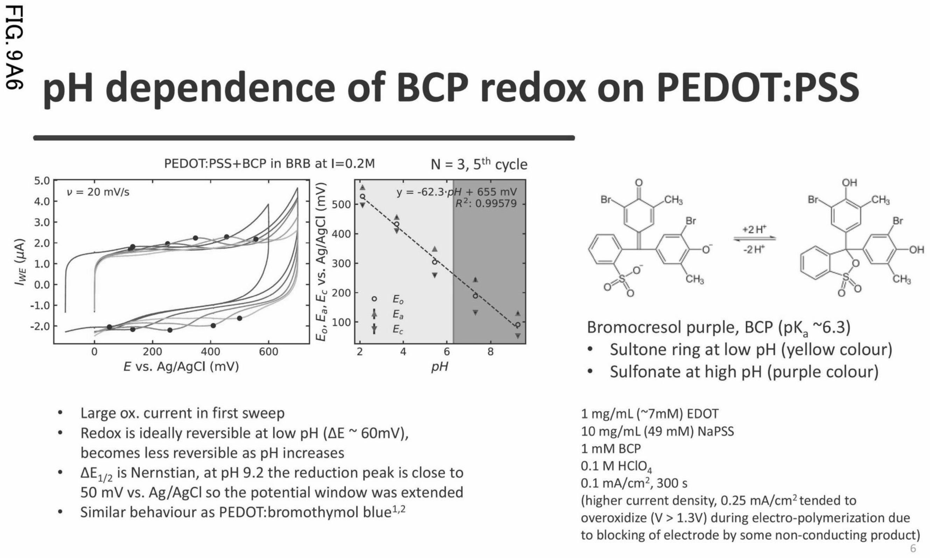

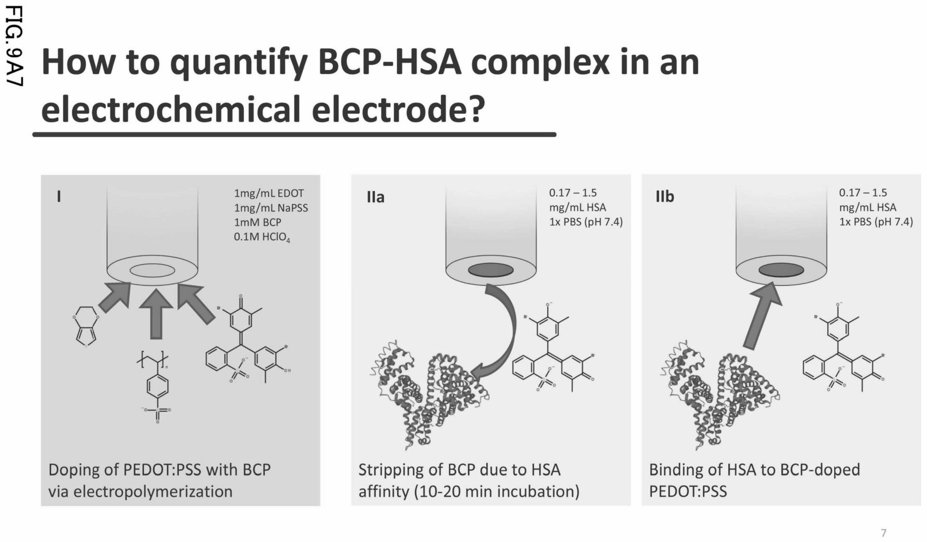

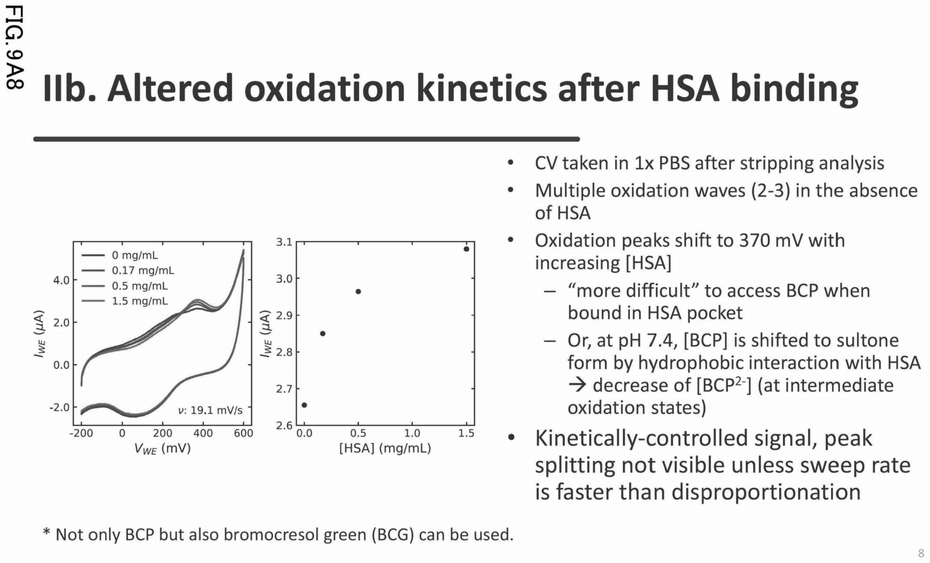

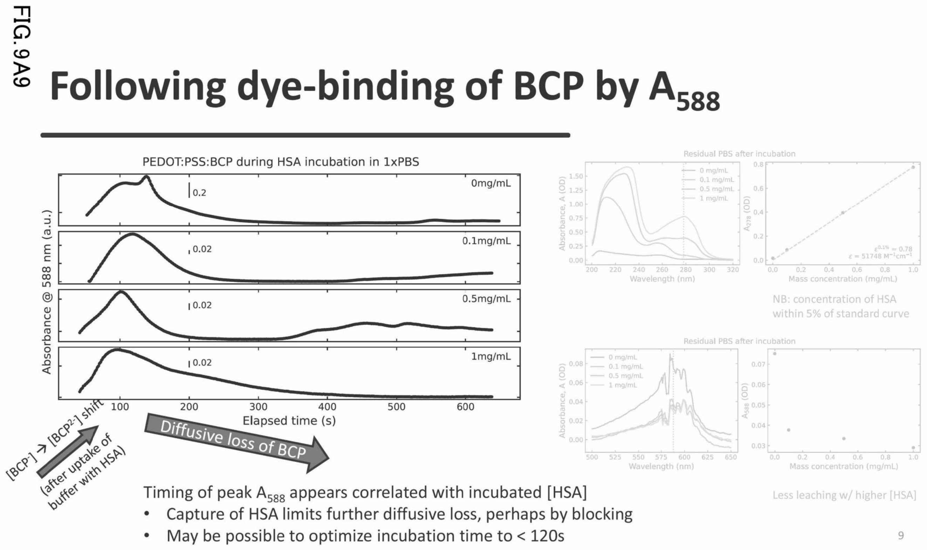

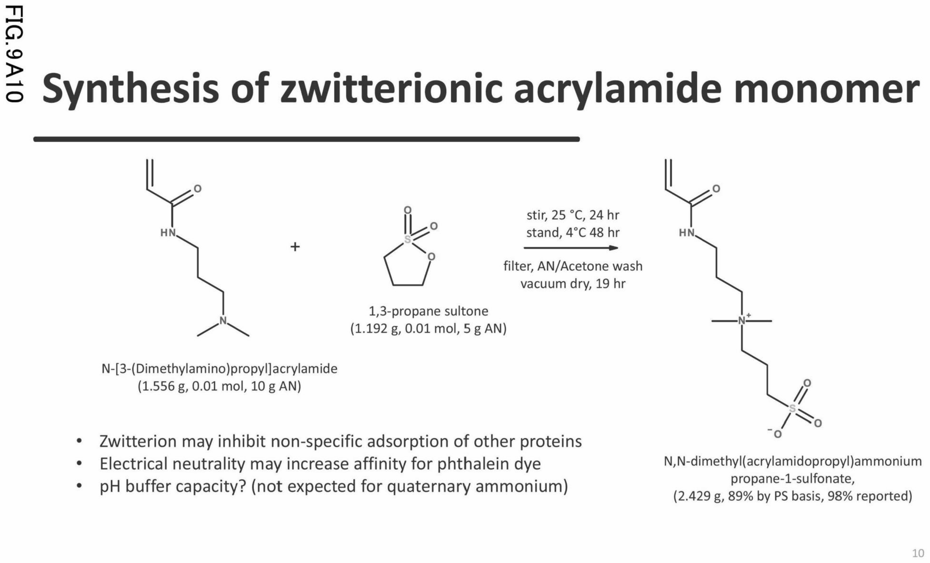

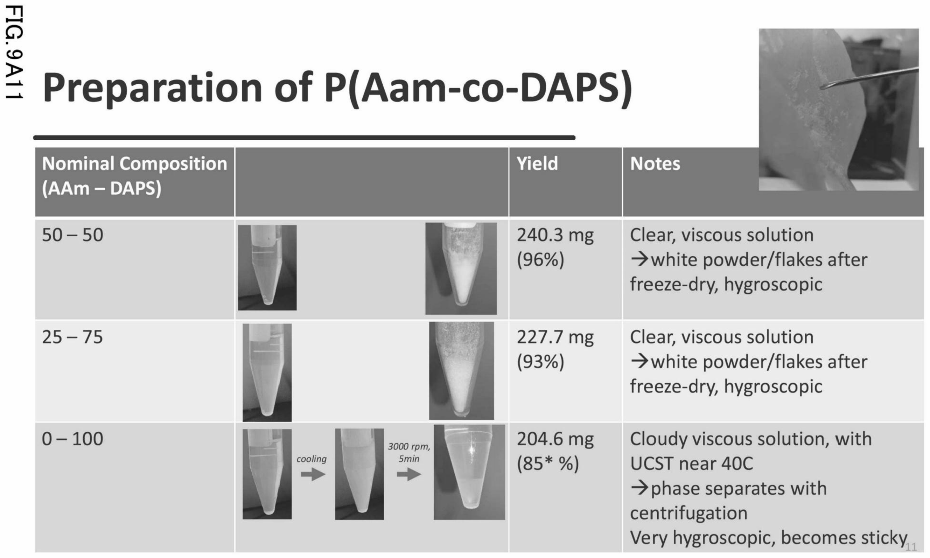

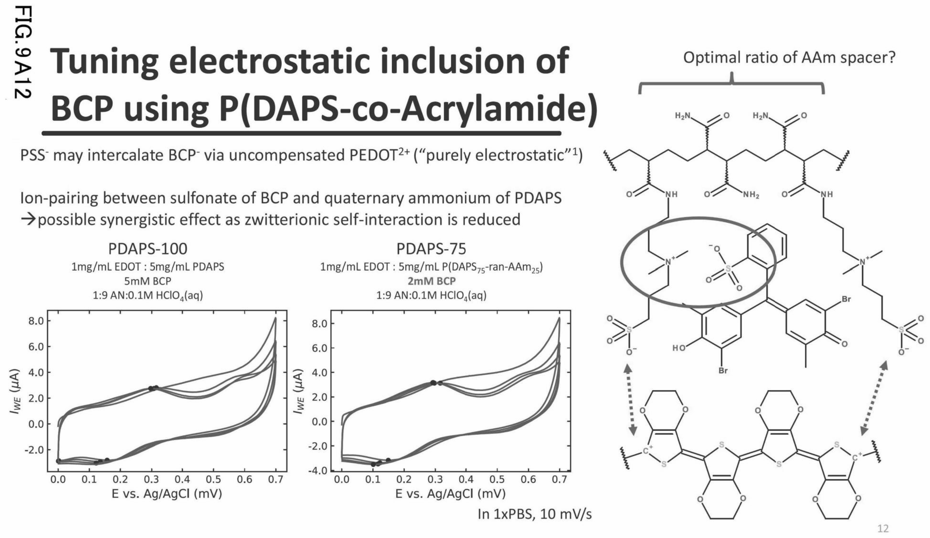

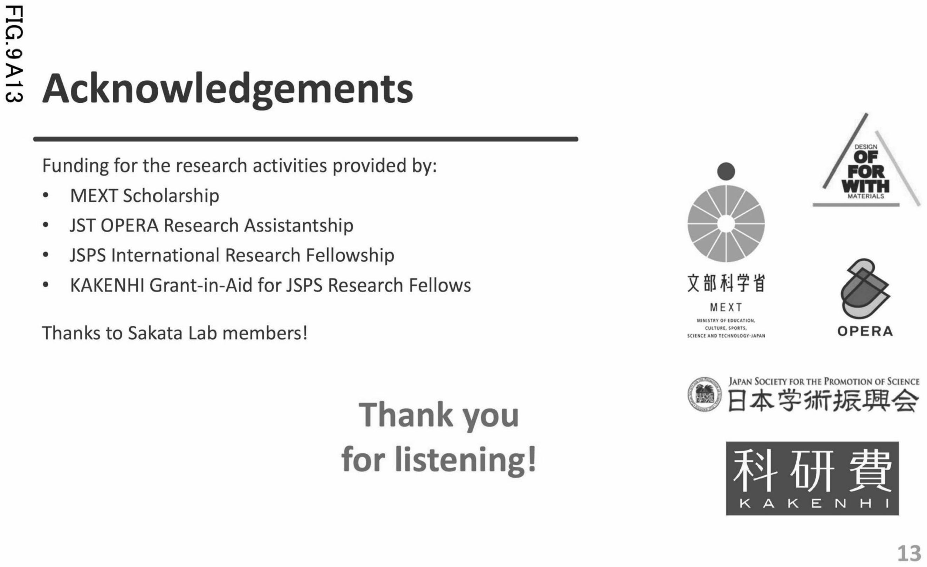

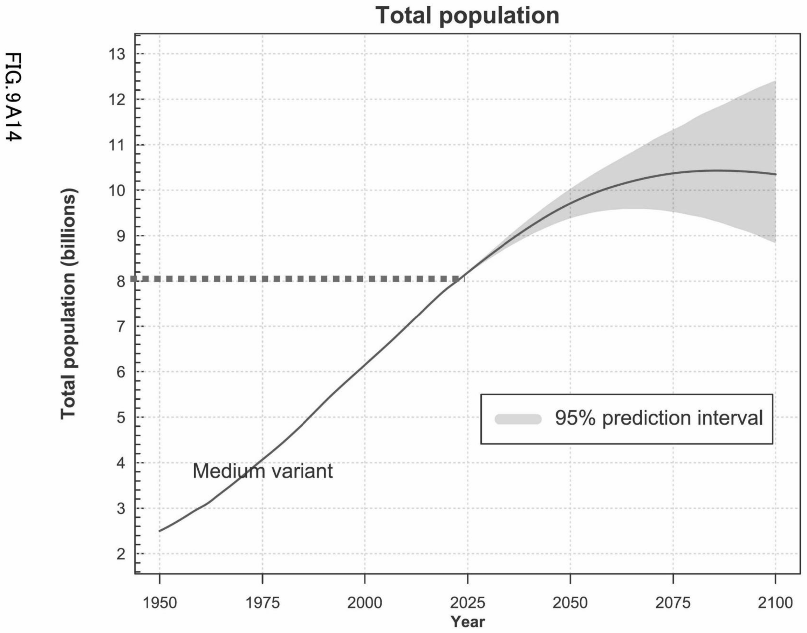

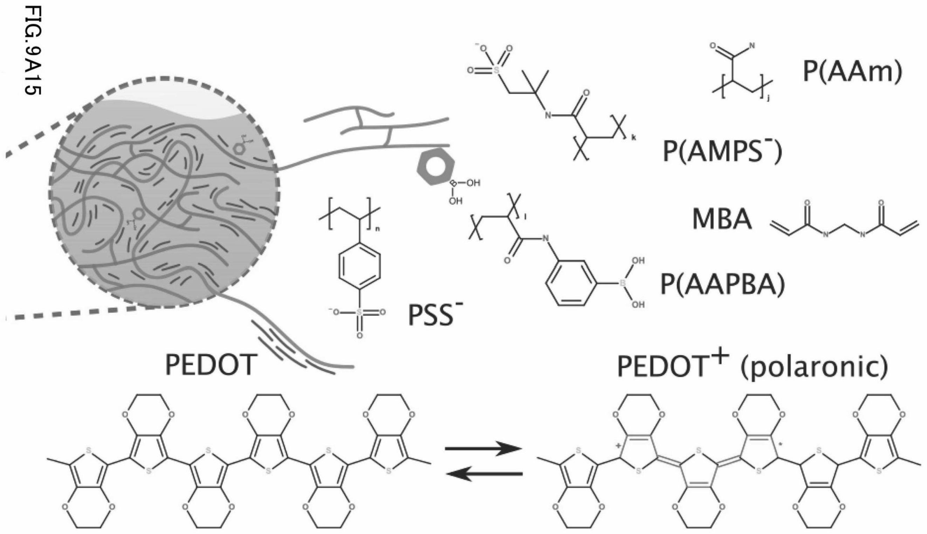

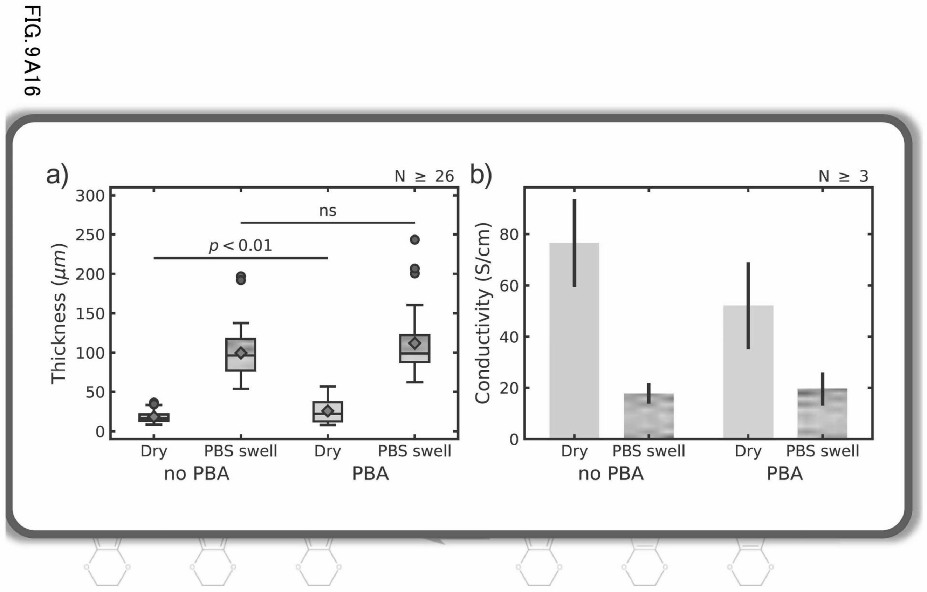

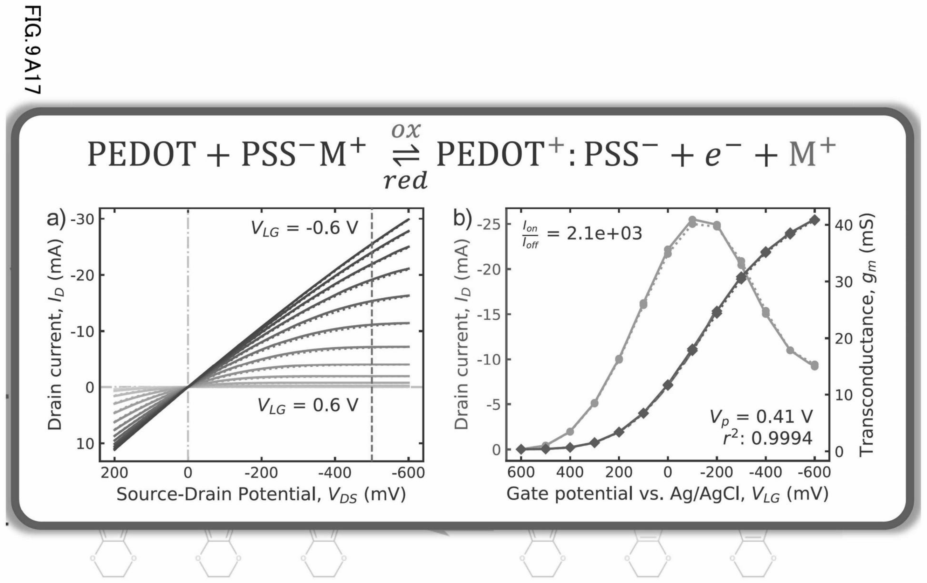

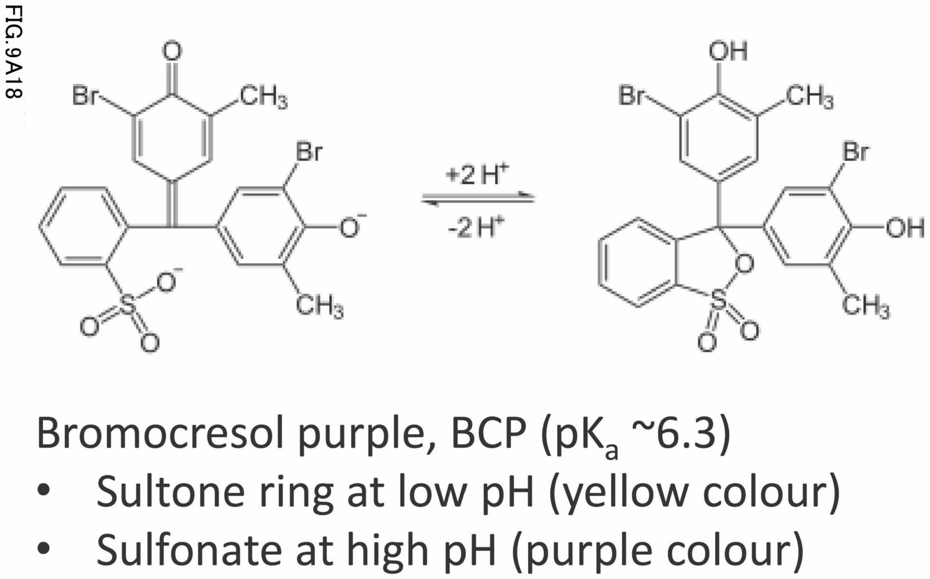

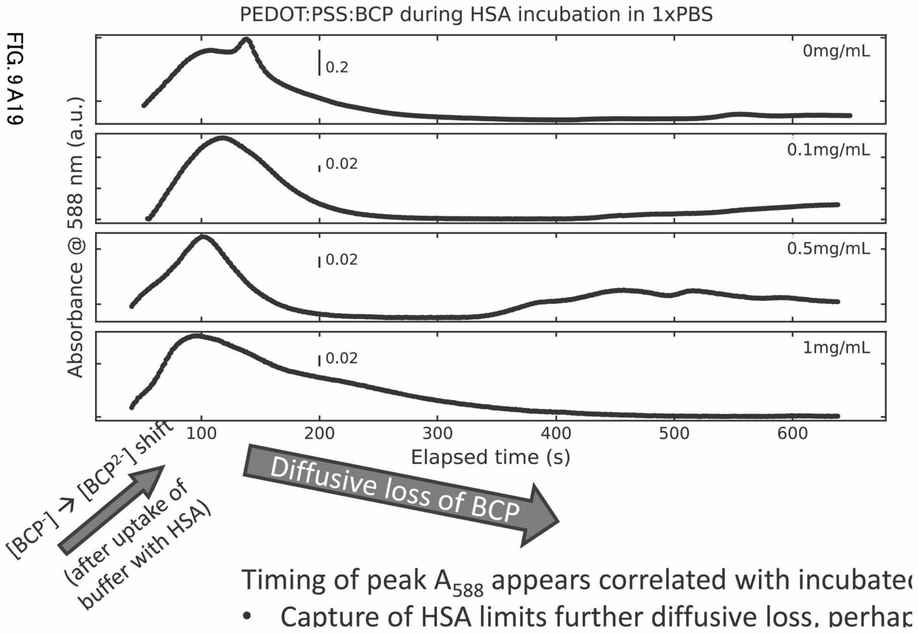

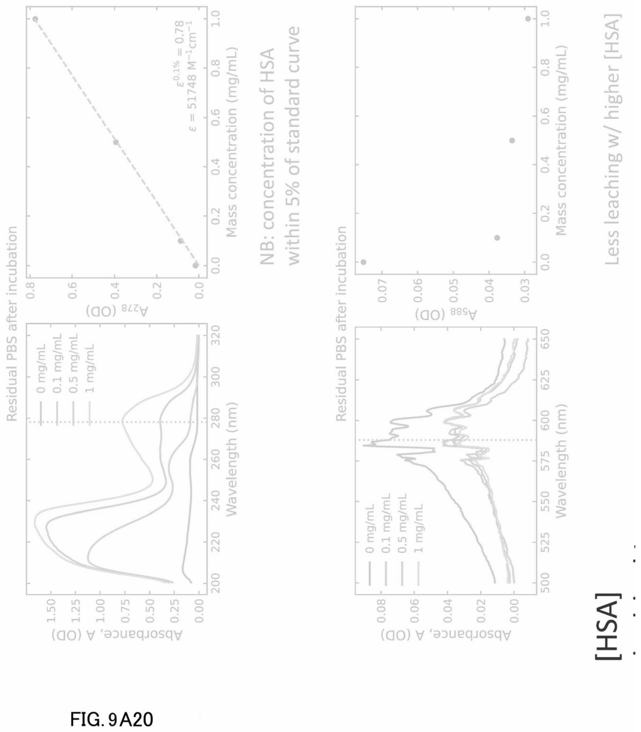

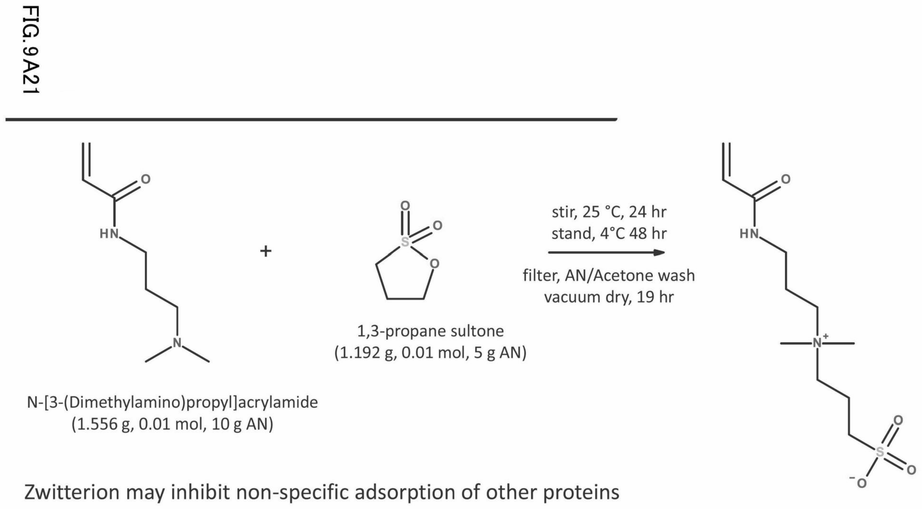

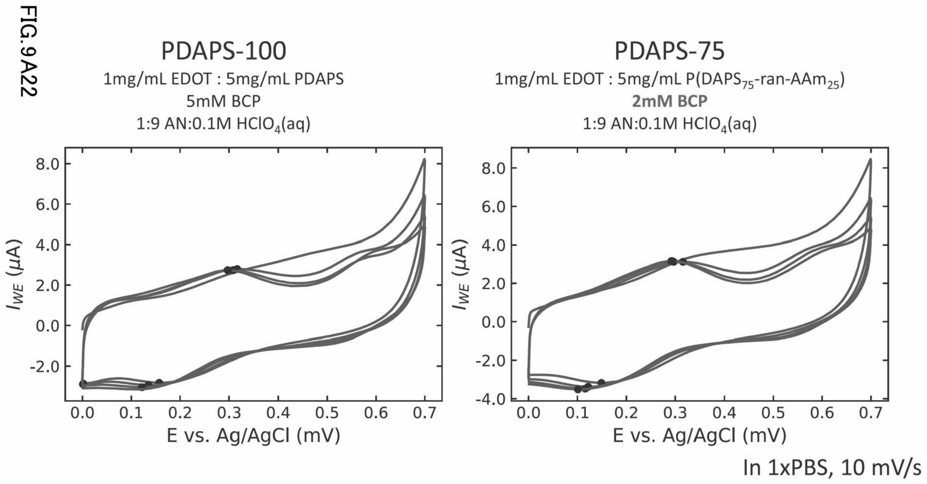

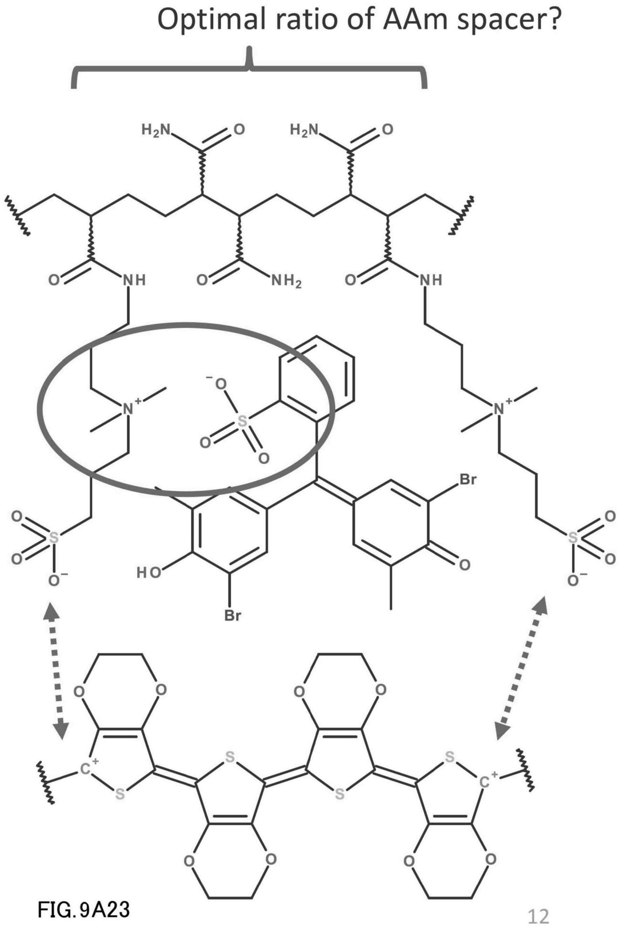

FIG. 9A2 is a description regarding evaluation of a PEDOT:BCP electrochemical electrode in human serum albumin measurement by a dye-binding method (p2).
FIG. 9A3 is a description regarding evaluation of a PEDOT:BCP electrochemical electrode in human serum albumin measurement by a dye-binding method (p3).
FIG. 9A4 is a description regarding evaluation of a PEDOT:BCP electrochemical electrode in human serum albumin measurement by a dye-binding method (p4).
FIG. 9A5 is a description regarding evaluation of a PEDOT:BCP electrochemical electrode in human serum albumin measurement by a dye-binding method (p5).
FIG. 9A6 is a description regarding evaluation of a PEDOT:BCP electrochemical electrode in human serum albumin measurement by a dye-binding method (p6).
FIG. 9A7 is a description regarding evaluation of a PEDOT:BCP electrochemical electrode in human serum albumin measurement by a dye-binding method (p7).
FIG. 9A8 is a description regarding evaluation of a PEDOT:BCP electrochemical electrode in human serum albumin measurement by a dye-binding method (p8).
FIG. 9A9 is a description regarding evaluation of a PEDOT:BCP electrochemical electrode in human serum albumin measurement by a dye-binding method (p9).
FIG. 9A10 is a description regarding evaluation of a PEDOT:BCP electrochemical electrode in human serum albumin measurement by a dye-binding method (p10).
FIG. 9A11 is a description regarding evaluation of a PEDOT:BCP electrochemical electrode in human serum albumin measurement by a dye-binding method (p11).
FIG. 9A12 is a description regarding evaluation of a PEDOT:BCP electrochemical electrode in human serum albumin measurement by a dye-binding method (p12).
FIG. 9A13 is a description regarding evaluation of a PEDOT:BCP electrochemical electrode in human serum albumin measurement by a dye-binding method (p13).
FIG. 9A14 is a description regarding evaluation of a PEDOT:BCP electrochemical electrode in human serum albumin measurement by a dye-binding method (p14).
FIG. 9A15 is a description regarding evaluation of a PEDOT:BCP electrochemical electrode in human serum albumin measurement by a dye-binding method (p15).
FIG. 9A16 is a description regarding evaluation of a PEDOT:BCP electrochemical electrode in human serum albumin measurement by a dye-binding method (p16).
FIG. 9A17 is a description regarding evaluation of a PEDOT:BCP electrochemical electrode in human serum albumin measurement by a dye-binding method (p17).
FIG. 9A18 is a description regarding evaluation of a PEDOT:BCP electrochemical electrode in human serum albumin measurement by a dye-binding method (p18).
FIG. 9A19 is a description regarding evaluation of a PEDOT:BCP electrochemical electrode in human serum albumin measurement by a dye-binding method (p19).
FIG. 9A20 is a description regarding evaluation of a PEDOT:BCP electrochemical electrode in human serum albumin measurement by a dye-binding method (p20).
FIG. 9A21 is a description regarding evaluation of a PEDOT:BCP electrochemical electrode in human serum albumin measurement by a dye-binding method (p21).
FIG. 9A22 is a description regarding evaluation of a PEDOT:BCP electrochemical electrode in human serum albumin measurement by a dye-binding method (p22).
FIG. 9A23 is a description regarding evaluation of a PEDOT:BCP electrochemical electrode in human serum albumin measurement by a dye-binding method (p23).

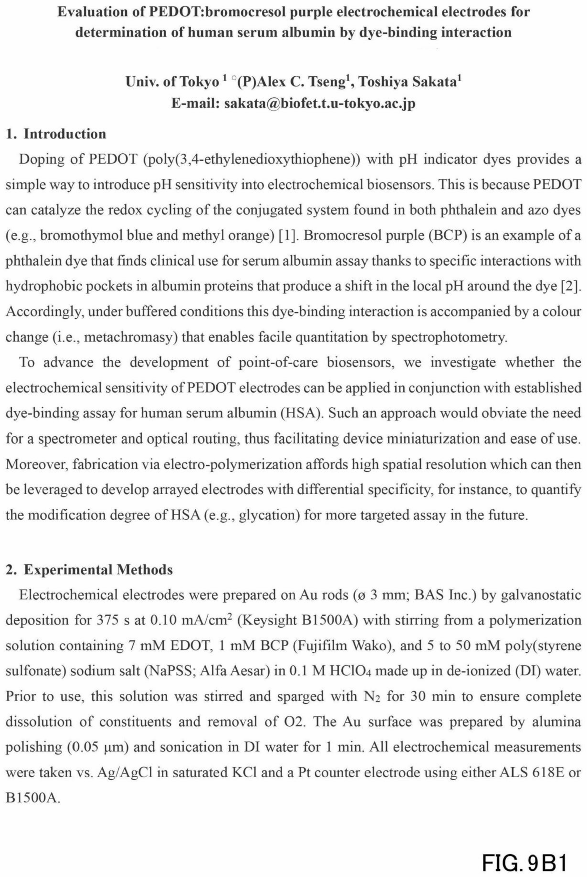

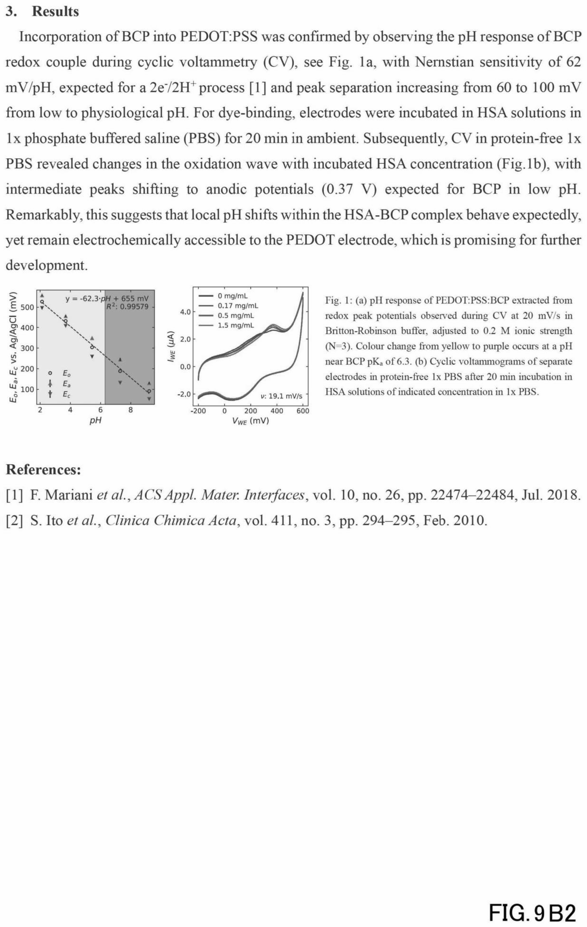

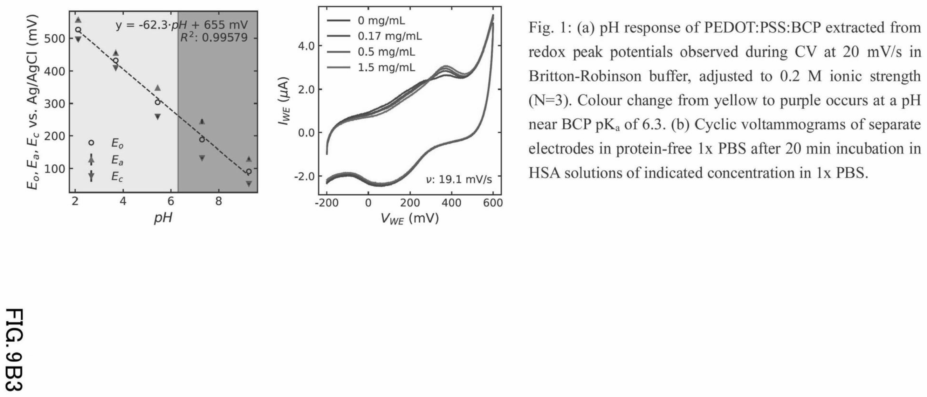

FIG. 9B1 is a description regarding evaluation of a PEDOT:BCP electrochemical electrode in human serum albumin measurement by a dye-binding method (Abst p1).
FIG. 9B2 is a description regarding evaluation of a PEDOT:BCP electrochemical electrode in human serum albumin measurement by a dye-binding method (Abst p2).
FIG. 9B3 is a description regarding evaluation of a PEDOT:BCP electrochemical electrode in human serum albumin measurement by a dye-binding method (Abst p3).

### DESCRIPTION OF EMBODIMENTS

As used herein, the term "conductive polymer" should be understood as a general scientific term and refers to a polymer that has electrical conductivity and whose electrical conductivity can change upon appropriate doping. Examples of the "conductive polymer" include, but are not limited to, poly(3,4-ethylenedioxythiophene) (PEDOT), polyaniline (PANI), polythiophene (PT), polypyrrole (PPy), polyfluorene (PF), poly(p-phenylene vinylene) (PPV), and poly(methyl methacrylate) (PMMA).

As used herein, the term "molecular recognition pH indicator" should be understood as a general scientific term and refers to a substance having an ability to recognize a target biomolecule and having a function as a pH indicator. Generally, the pH indicator is a small molecule.

The molecular recognition pH indicator may be selected according to the target biomolecule. Examples of combinations of the target biomolecule and the molecular recognition pH indicator that recognizes the target biomolecule are shown in Table 1.

**[Table 1]**

| Target Biomolecule | Molecular Recognition pH Indicator |
|---|---|
| Albumin | Bromocresol Purple (BCP) |
| Albumin | Bromocresol Green (BCG) |
| Glucose | Ferrocene derivative |
| Cholesterol | Cholesterol esterase |
| Lactate | NADH-dependent enzyme |
| Urea | Urease |
| Creatinine | Picric acid |
| Amino acid (e.g., Glutamic acid) | Glutamate dehydrogenase |
| ATP | Luciferase |
| Bilirubin | Barbituric acid |

In some embodiments, the target biomolecule is albumin, and the molecular recognition pH indicator thereof may be Bromocresol Purple (BCP) or Bromocresol Green (BCG). For example, the molecular recognition pH indicator may be BCP.

The molecular recognition pH indicator may be disposed inside a network of the conductive polymer. For example, the molecular recognition pH indicator may be mixed into a monomer at the start of or prior to polymerization. Thereby, for example, the molecular recognition pH indicator can be uniformly disposed inside the network of the conductive polymer, becomes easier to stay inside the network of the conductive polymer, and/or a probability or speed of detachment from the network of the conductive polymer can be reduced.

In some embodiments, another polymer or dopant may be disposed within the network of the conductive polymer. Examples of combinations of the conductive polymer and the dopant are shown in Table 2.

**[Table 2]**

| Conductive Polymer | Substance Functioning as Dopant |
|---|---|
| Poly(3,4-ethylenedioxythiophene) (PEDOT) | Polystyrene sulfonic acid (PSS) |
| Polyaniline (PANI) | Camphorsulfonic acid (CSA), p-Toluenesulfonic acid (p-TSA) |
| Polythiophene (PT) | Ferric chloride (FeCl₃), Bromochloromethane (BCM) |
| Polypyrrole (PPy) | p-Toluenesulfonic acid (p-TSA), Naphthalenesulfonic acid (NSA) |
| Polyfluorene (PF) | Trifluoromethanesulfonic acid (TFMSA), Sulfuric acid (H₂SO₄) |
| Poly(p-phenylene vinylene) (PPV) | Ferric chloride (FeCl₃), Iodine (I₂) |
| Poly(methyl methacrylate) (PMMA) | Lithium salt (e.g., LiClO₄), Aluminum salt (e.g., AlCl₃) |

In some embodiments, the conductive polymer is PEDOT, and polystyrene sulfonic acid (PSS) may be disposed within the network of PEDOT. In some embodiments, the conductive polymer is PEDOT, and PSS and BCP may be disposed within the network of PEDOT.

The conductive polymer may be prepared by polymerizing a monomer. In some embodiments, a layer comprising the conductive polymer and the dopant may be formed by providing at least a monomer for the conductive polymer and polymerizing the monomer. The polymerization may be performed by applying a solution containing the monomer to a desired position on a substrate surface and leaving the solution at room temperature. For example, a conductive polymer member or a conductive polymer layer may be formed by applying a polymerization solution to a predetermined surface of an electrode, a sensor substrate, or the like, and polymerizing the monomer contained in the polymerization solution. For example, the conductive polymer member or conductive polymer layer generated by polymerization may be disposed on a predetermined surface of an electrode, a sensor substrate, or the like.

The polymerization may be electric field polymerization.

The polymerization may be electropolymerization. For example, the conductive polymer can be formed on an electrode surface by applying a solution containing the monomer to an electrode surface and applying a potential to the electrode. For example, the conductive polymer can be formed as a channel connecting a drain electrode and a source electrode by applying a solution containing the monomer to surfaces of the drain electrode, the source electrode, and an insulator therebetween for a transistor, and applying an electric field to the drain electrode and the source electrode.

As used herein, the term "polymerization solution" generally refers to a solution containing a monomer to be polymerized. The polymerization solution may further contain an initiator, a catalyst, and other additives.

In some embodiments, the polymerization solution may contain a mixture of a dopant or a substance serving as a material therefore, in addition to the monomer for the conductive polymer. For example, when the conductive polymer comprises PEDOT and the dopant comprises BCP or BCG, the polymerization solution may contain 3,4-ethylenedioxythiophene (EDOT) and BCP or BCG. EDOT polymerizes to form PEDOT. In some embodiments, BCP or BCG may exist as a single molecule in the network of PEDOT. In some embodiments, BCP or BCG may polymerize and exist in the network of PEDOT. For example, BCP or BCG may be polymerized simultaneously with or separately from a polymerization process of EDOT. Poly-bromocresol purple or a BCP-containing polymer may be mixed in the network of the conductive polymer.

In some embodiments, the conductive polymer may be formed by polymerization, and thereafter, the dopant may be introduced into the network of the conductive polymer. For example, the formed network of PEDOT may be impregnated with BCP or BCG by immersing the formed network of PEDOT in a solution of BCP or BCG.

In some embodiments, the target biomolecule may be provided by being contained in a liquid (solution). The liquid may be a body fluid secreted from a subject, or may be a liquid other than a body fluid. The liquid other than a body fluid may be a liquid adhering to the subject, or may be a liquid not adhering to the subject. The liquid not adhering to the subject may be a liquid accommodated in the subject.

The provided liquid may contain the target biomolecule, may have a possibility of containing the target biomolecule, or may be a reference liquid used for measuring the target biomolecule. Hereinafter, a liquid (or solution) containing the target biomolecule may refer to these cases.

The liquid containing the target biomolecule may be a solution. The liquid may be a body fluid, a solution derived from a body fluid, or a diluted solution of a body fluid. The liquid may be a non-body fluid (non-body fluid-derived) solution, or a mixture of a body fluid or a body fluid-derived solution and a non-body fluid-derived solution. The solution may be a solution used for sample measurement, or may be a solution used for calibration measurement. For example, the solution may be a standard solution or a calibration solution. A sample to be measured may be a specimen.

The body fluid may be lymph, a tissue fluid such as interstitial fluid, intercellular fluid, stromal fluid, and the like, or may be body cavity fluid, serous cavity fluid, pleural effusion, ascites, pericardial fluid, cerebrospinal fluid (CSF), synovial fluid, or aqueous humor. The body fluid may be digestive fluid such as saliva, gastric juice, bile, pancreatic juice, or intestinal juice, or may be sweat, tears, nasal discharge, urine, semen, vaginal fluid, amniotic fluid, or milk. The body fluid may be animal body fluid or human body fluid. The body fluid may be a solution. The solution may contain a physiological buffer such as phosphate buffered saline (PBS), N-tris(hydroxymethyl)methyl-2-aminoethanesulfonic acid buffer (TES), or hydroxyethylpiperazineethanesulfonic acid buffer (HEPES), which contains a substance to be measured. The solution is not particularly limited as long as it contains the substance to be measured.

In some embodiments, the body fluid may be blood. In some embodiments, blood may be collected. For example, blood may be collected simultaneously with bleeding by puncture. For example, blood may be aspirated by inserting a needle. In some embodiments, a puncture tool (e.g., a needle, an injection needle, etc.; the same applies hereinafter) may be disposed at a tip of a capillary. In some embodiments, a capillary may be formed as a puncture tool.

In some embodiments, the subject may include a human, or may be a human. In some embodiments, the subject may include a non-human animal, or may be a non-human animal. The non-human animal may include a mammal, or may be a mammal. The non-human animal may be, for example, but not limited to, a working animal, a domestic animal, a pet animal, or a wild animal.

As used herein, the term "electrode" is a broad term and should be given its ordinary and customary meaning to those skilled in the art and should not be limited to a special or specific meaning. In some embodiments, the electrode may be used for electrochemical measurement. The electrode may be formed of a conductive material. The electrode may be formed of a metal. The electrode may be formed including, for example, but not limited to, a metal material such as gold (Au), platinum (Pt), or palladium (Pd). The electrode may be formed of a non-metallic material. For example, but not limited to, the electrode may be formed of carbon (C). The electrode may be formed of graphene, carbon nanotube, or the like.

The electrode may be connected to, or may be configured to be connected to, a measuring instrument, a measuring device, or a measuring element.

In some embodiments, the electrode may be connected to an impedance measuring instrument. In some embodiments, an electrical signal at the electrode may be detected or analyzed using electrochemical impedance spectroscopy (EIS). Thereby, a Cole-Cole plot or a Nyquist plot may be generated. A capacitive component of a measurement system may be determined or analyzed. In some embodiments, an electrical signal at the electrode may be detected or analyzed using cyclic voltammetry (CV).

In some embodiments, a current at a potential corresponding to an oxidation-reduction reaction of the conductive polymer may be measured. In that case, a current measuring device may be used. The amount of the target biomolecule may be determined from a peak value of the current at the potential corresponding to the oxidation-reduction reaction, or a difference of the peak value from a background.

In some embodiments, an electrode in the vicinity of an aptamer may be used as a working electrode (WE). In addition, a counter electrode (CE) and further a reference electrode (RE) may be disposed.

In some embodiments, the electrode may be connected to an ammeter, an amperometer, or the like. An electrical signal at the electrode may be measured or analyzed amperometrically.

In some embodiments, the electrode may be connected to a voltmeter, a potentiometer, a transistor (e.g., a field-effect transistor), or the like. An electrical signal at the electrode may be measured or analyzed potentiometrically.

In some embodiments, the electrode may be an electrode of a transistor. For example, the electrode may be an electrode of a so-called biotransistor, or may be an electrode of a so-called FET (field-effect transistor) biosensor. The electrode may be a drain and/or a source of a transistor.

### Example 1

An electrochemical electrode was fabricated on an end surface of a gold (Au) rod (diameter 3 mm, BAS Inc.). The Au end surface was polished with alumina (particle size 0.05 µm) and ultrasonically cleaned in DI water for 1 minute. Galvanostatic deposition was performed in a polymerization solution. The polymerization solution contained 7 mM EDOT, 1 mM BCP (Fujifilm Wako), and 5 to 50 mM poly(styrene sulfonic acid) sodium salt (NaPSS) (Alfa Aesar), and was prepared with 0.1 M perchloric acid (HClO₄) in deionized water (DI water). Prior to use, this solution was bubbled with nitrogen (N₂) gas for 30 minutes while stirring to completely dissolve components and remove oxygen (O₂). The galvanostatic deposition was performed for 375 seconds at a current density of 0.10 mA/cm² while stirring the solution (Keysight B1500A). Thereby, a composition in a film can be made uniform. A PEDOT film having a thickness of about 100 nm and doped with BCP and PSS was formed on the gold electrode surface.

For all electrochemical measurements, an Ag/AgCl reference electrode in saturated KCl and a Pt counter electrode, and an electrochemical analyzer (ALS 618E, BAS Inc., or B1500A, Keysight) were used. HSA solutions were adjusted to concentrations of 0 mg/mL, 0.17 mg/mL, 0.5 mg/mL, and 1.5 mg/mL using 1× phosphate buffered saline (PBS).

Incorporation of BCP into the network of PEDOT was confirmed by observing a pH response of a BCP redox couple during cyclic voltammetry (CV) (not shown). An HSA solution was introduced into 1× phosphate buffered saline (PBS) and incubated at room temperature for 20 minutes. Thereafter, as a pre-measurement treatment, a voltage up to 0.6 V was applied to the Ag/AgCl reference electrode, and a current of 28 µA/cm² was passed.

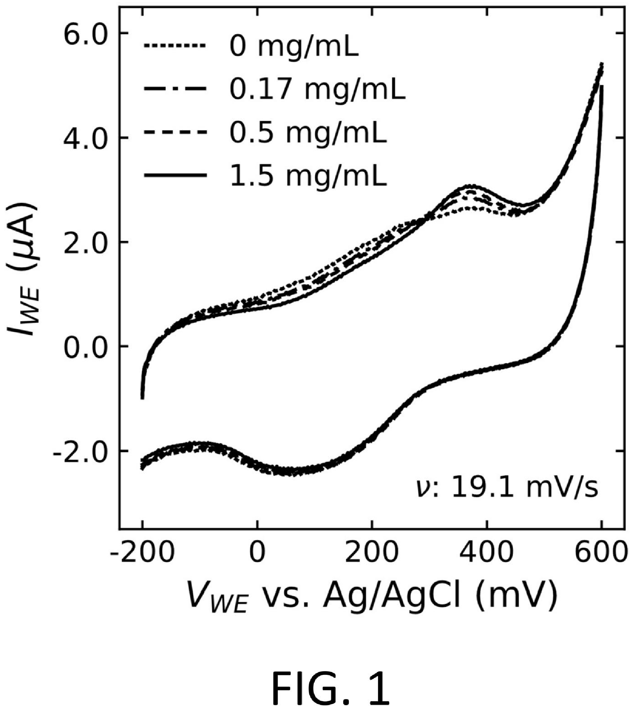

FIG. 1 shows CV characteristics of the HSA solutions of respective concentrations (horizontal axis: potential applied to the working electrode relative to the Ag/AgCl reference electrode (V_{WE}), vertical axis: current flowing through the working electrode (I_{WE})). An oxidation peak of an oxidation-reduction reaction of BCP incorporated in the PEDOT network was observed at 370 mV. It was observed that a height of this oxidation peak changed with the HSA concentration of the HSA solution.

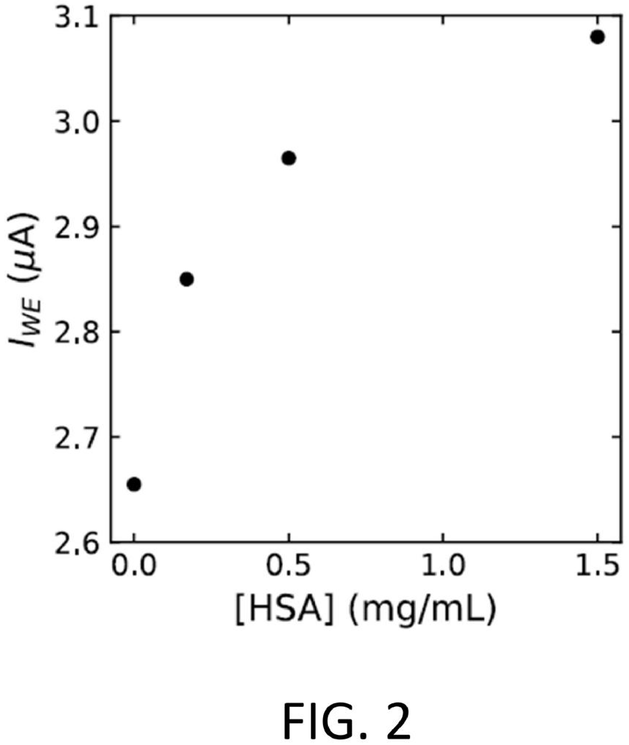

FIG. 2 shows a working electrode current (peak current Ipeak) at 370 mV of the CV characteristics with respect to the HSA concentration. From this, it was revealed that the peak current at the working electrode monotonically increased with the HSA concentration. This result indicates that the HSA-BCP complex in the PEDOT network can be electrochemically and quantitatively detected.

As one scientific consideration, it is considered that BCP binds to albumin to undergo a structural change and enters a reduced state, and the albumin-BCP complex becomes easier to oxidize. An anion component in BCP can function as a dopant for PEDOT. It is considered that the variation in the height of the oxidation peak in the CV characteristics of PEDOT containing BCP reflects a change in oxidation-reduction of BCP due to the formation of the albumin-BCP complex or conductivity of PEDOT. However, this mechanism is one of hypotheses that the inventors can propose at this stage, and the content of the present disclosure should not be interpreted as being limited to this scientific consideration. This description does not exclude interpretation of the present disclosure based on other scientific considerations or mechanisms.

BCP is a small molecule compared to a polymer. Therefore, it was not known that a sufficient amount of BCP for PEDOT to electrically sense the reaction with the HSA-BCP complex stays in the vicinity of PEDOT molecules or inside the network of PEDOT. Furthermore, it was not known, at least to the knowledge of the inventors, that BCP functions as a dopant for a conductive polymer such as PEDOT. The inventors have found for the first time in the world that the HSA-BCP complex is electrochemically and quantitatively detectable by PEDOT.

Those skilled in the art will recognize that this result holds true not only for the system of HSA and PEDOT:BCP but also for a broader concept. That is, the present disclosure also includes that a reaction between a biomolecule and a molecular recognition pH indicator that recognizes the biomolecule and can function as a dopant affects an electrical property of a conductive polymer, and that the biomolecule can be detected or quantified based on the electrical property.

The electrical property of the conductive polymer can be measured by various methods. In some embodiments, cyclic voltammetry may be used. In some embodiments, an oxidation-reduction reaction in the vicinity of an electrode on a surface of which the conductive polymer is disposed may be measured. For example, a height of an oxidation-reduction peak (current value) may be measured. In some embodiments, conductivity of the conductive polymer may be measured. For example, a transistor having the conductive polymer containing the molecular recognition pH indicator as a channel may be configured. A current of the channel (drain current) generated by the reaction between the target biomolecule and the molecular recognition pH indicator may be measured.

### Embodiment 1-1

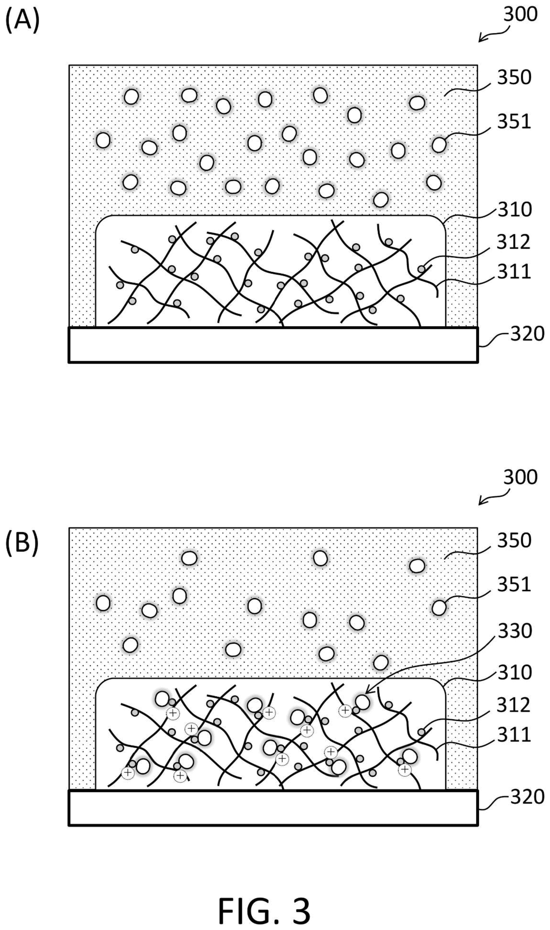

FIG. 3 shows a schematic cross-sectional view of a device 300 for detecting a biomolecule according to an embodiment. The device 300 comprises a recognition unit 310 and a transducer 320.

The recognition unit 310 includes a network of a conductive polymer 311 and a molecular recognition pH indicator 312 that recognizes a target biomolecule 351. The molecular recognition pH indicator 312 is disposed within the network of the conductive polymer 311 such that it can transmit an electrical signal generated by recognizing the target biomolecule 351 to the conductive polymer 311.

In FIG. 3 and the following figures, the molecular recognition pH indicators 312, 412, and 512 are disposed in the vicinity of the conductive polymers 311, 411, and 511. However, these figures are schematic, and locations, modes, etc., of arrangement of respective molecules should not be construed as being limited thereto.

The device 300 may have a fluid chamber (not shown) capable of accommodating the recognition unit 310 therein and receiving a fluid. A solution 350 containing the target biomolecule 351 is introduced into the fluid chamber of the device 300 (FIG. 3A). The solution 350 comes into contact with a surface of the recognition unit 310.

The target biomolecule 351 in the solution 350 flows into the network of the conductive polymer 311. The target biomolecule 351 is captured by the molecular recognition pH indicator 312 to form a complex 330. This complex 330 functions as a dopant and generates a positive charge in the conductive polymer 311 (plus sign in the figure). Thereby, the electrical property or conductive property of the conductive polymer 311 is changed.

The transducer 320 is disposed in the vicinity of the recognition unit 310 and is configured to electrochemically detect a chemical reaction in the recognition unit 310. The transducer 320 is configured to electrically detect the electrical property of the conductive polymer 311 or a change thereof.

The transducer 320 may be connected to an electronic device or a circuit, such as a computer, that analyzes a signal output by the transducer 320 (not shown).

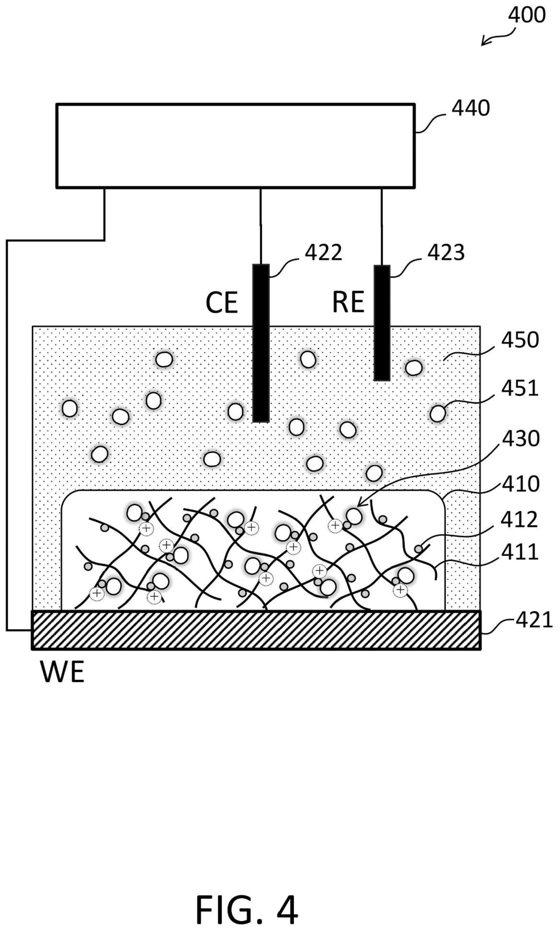

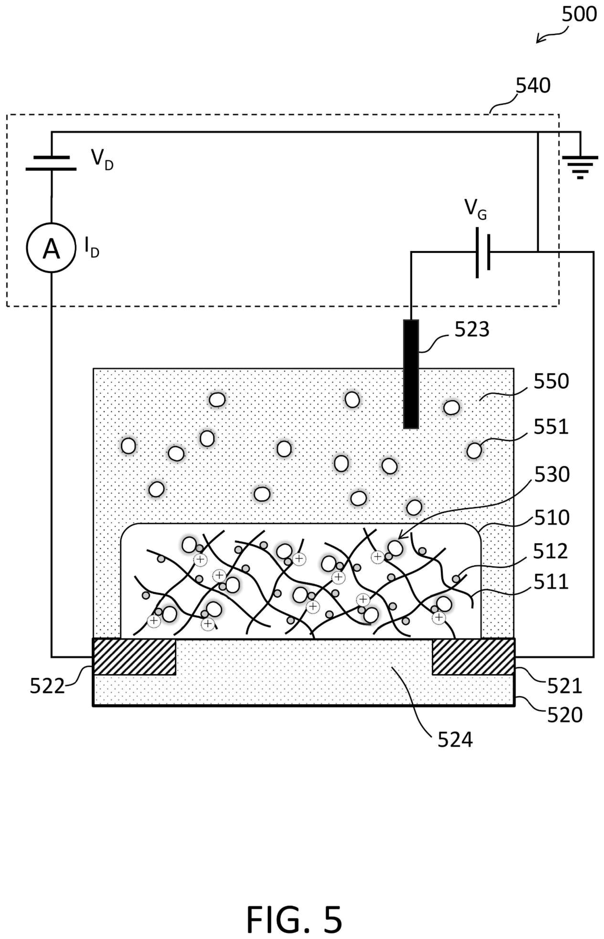

With reference to FIG. 4 and FIG. 5, more specific embodiments of this embodiment will be described.

### Embodiment 1-2: CV Measurement

FIG. 4 shows a schematic cross-sectional view of a device 400 for detecting a biomolecule by cyclic voltammetry (CV method) according to an embodiment. The device 400 comprises a recognition unit 410 and an electrode 421 as a transducer.

The recognition unit 410 includes a network of a conductive polymer 411 and a molecular recognition pH indicator 412 that recognizes a target biomolecule 451. The molecular recognition pH indicator 412 is disposed within the network of the conductive polymer 411 such that it can transmit an electrical signal generated by recognizing the target biomolecule 451 to the conductive polymer 411.

The device 400 may have a fluid chamber (not shown) capable of accommodating the recognition unit 410 therein and receiving a fluid. A solution 450 containing the target biomolecule 451 is introduced into the fluid chamber of the device 400 (FIG. 4A). The solution 450 comes into contact with a surface of the recognition unit 410.

The target biomolecule 451 in the solution 450 flows into the network of the conductive polymer 411. The target biomolecule 451 is captured by the molecular recognition pH indicator 412 to form a complex 430.

An electrode 420 is disposed in the vicinity of the recognition unit 410 and is configured to electrochemically detect a chemical reaction in the recognition unit 410. In FIG. 4, the electrode 420 has a flat surface, and the conductive polymer 411 is disposed in layers on the surface. In FIG. 4, a three-electrode method is used. A counter electrode (CE) 423 and a reference electrode (RE) 423 are disposed so as to be in contact with the solution 450. The electrode 420 functions as a working electrode (WE).

Thereby, an oxidation-reduction reaction of the conductive polymer 411 due to the formation of the complex 430 of the target biomolecule 451 and the molecular recognition pH indicator 412 can be subjected to CV measurement using three electrodes.

In FIG. 4, the device 400 has an analyzer 440 connected to the working electrode (WE) 421, the counter electrode (CE) 422, and the reference electrode (RE) 423. The analyzer 440 may be a potentiostat, a galvanostat, or a potentiogalvanostat. In some embodiments, the analyzer 440 may be included in the device 400. In some embodiments, the analyzer 440 may be externally attached to the device 400 or configured to be connected to the device 400.

In some embodiments, a peak current at an oxidation-reduction potential (oxidation-reduction peak current) may be measured instead of CV. For that purpose, a current measuring device may be used.

### Embodiment 1-3: Transistor Measurement

FIG. 5 shows a schematic cross-sectional view of a device 500 for detecting a biomolecule using a transistor configuration according to an embodiment. The device 500 comprises a recognition unit 510, and a source electrode 521, a drain electrode 522, and a gate electrode 523 as transducers.

The recognition unit 510 includes a network of a conductive polymer 511 and a molecular recognition pH indicator 512 that recognizes a target biomolecule 551. The molecular recognition pH indicator 512 is disposed within the network of the conductive polymer 511 such that it can transmit an electrical signal generated by recognizing the target biomolecule 551 to the conductive polymer 511.

The device 500 may have a fluid chamber (not shown) capable of accommodating the recognition unit 510 therein and receiving a fluid. A solution 550 containing the target biomolecule 551 is introduced into the fluid chamber of the device 500. The solution 550 comes into contact with a surface of the recognition unit 510.

The target biomolecule 551 in the solution 550 flows into the network of the conductive polymer 511. The target biomolecule 551 is captured by the molecular recognition pH indicator 512 to form a complex 530.

The source electrode 521 and the drain electrode 522 are formed on a surface of a substrate 520 and are insulated from each other by an insulator 524. The recognition unit 510 is formed on a surface of the insulator 524 between the source electrode 521 and the drain electrode 522, connected to the source electrode 521 at one end, and connected to the drain electrode 522 at the other end. Thereby, the recognition unit 510 functions as a channel between the source electrode 521 and the drain electrode 522. The channel electrode 523 is disposed so as to be in contact with the introduced solution 550.

Thereby, a change in conductivity of the conductive polymer 511 due to the formation of the complex 530 of the target biomolecule 551 and the molecular recognition pH indicator 512 can be measured by a biotransistor.

In FIG. 5, the device 500 has an analyzer 540 connected to the source electrode 521, the drain electrode 522, and the gate electrode 523. FIG. 5 shows a simplified circuit diagram as an example of the analyzer 540. However, this is merely an example, and other elements, circuits, etc. may be added, or other elements, circuits, etc. may be used instead of this circuit. The analyzer 540 may be a potentiostat, a galvanostat, or a potentiogalvanostat. In some embodiments, the analyzer 540 may be included in the device 500. In some embodiments, the analyzer 540 may be externally attached to the device 500 or configured to be connected to the device 500.

### Application to GA Level Measurement

The present disclosure also provides a method and a device for determining a GA level. The method or device for measuring an amount of albumin of the present disclosure can also be applied to determining a GA level. In some embodiments, an amount of albumin may be determined using the method or device of the present disclosure, an amount of glycated albumin may be determined, and a GA level, which is a ratio of the amount of glycated albumin to the total amount of albumin, may be determined from the determined amount of albumin and the amount of glycated albumin.

Examples of techniques for determining the amount of glycated albumin are shown below.

In some embodiments, the amount of glycated albumin may be measured using an enzymatic method. For example, a sample containing albumin that is partially glycated is provided, and this albumin is degraded using a protease to generate peptide fragments containing fructosyl lysine. Hydrogen peroxide is generated from these peptide fragments using fructosyl amino acid oxidase (FAOD). This hydrogen peroxide is quantified, and the amount of glycated albumin contained in the provided sample can be determined.

In some embodiments, the amount of glycated albumin may be measured using an aptamer. For example, a sample containing albumin that is partially glycated is provided, the glycated albumin in the sample is captured by an aptamer that specifically recognizes glycated albumin, the captured glycated albumin is quantified, and the amount of glycated albumin contained in the provided sample is determined. For example, a mediator may be used. An electrode capable of measuring the mediator is provided, and the aptamer is immobilized on a surface of this electrode. Depending on the amount of glycated albumin captured by the immobilized aptamer, a current flowing to the electrode due to the mediator decreases. By measuring this current, the amount of glycated albumin contained in the provided sample can be determined.

In some embodiments, the amount of glycated albumin may be measured using a conductive polymer such as PEDOT. For example, a substance having a phenylboronic acid group that recognizes glucose of glycated albumin may be mixed into PEDOT. For example, a polymer body of a double network in which PEDOT and a polymer having a phenylboronic acid group are mixed may be provided. A sample containing albumin that is partially glycated is introduced into this polymer body of the double network, and the phenylboronic acid group is caused to recognize glycated albumin. Depending on the amount of glycated albumin recognized by the phenylboronic acid group, an electrical property of PEDOT changes. By measuring this electrical property, the amount of glycated albumin contained in the provided sample can be determined.

In some embodiments, a device for measuring the total amount of albumin and a device for measuring the amount of glycated albumin may be provided individually. In some embodiments, a unit for measuring the total amount of albumin and a unit for measuring the amount of glycated albumin may be disposed and provided within one device.

### Embodiment 2-1: Flow Diversion

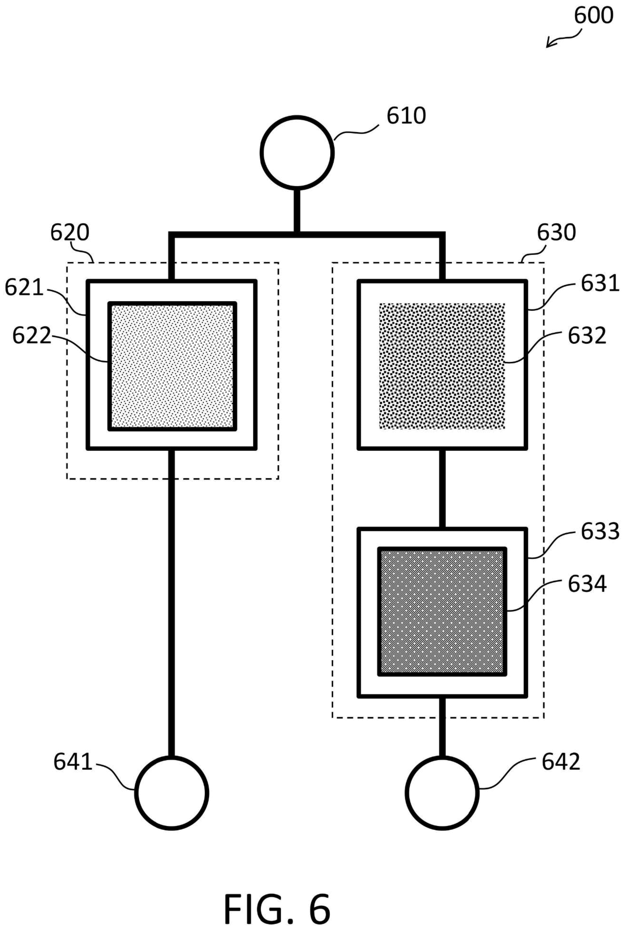

FIG. 6 schematically shows a configuration 600 of a fluidic device for determining a GA level according to an embodiment. The fluidic device 600 includes an albumin (Alb) sensor unit 620 using a conductive polymer and a glycated albumin (GA) sensor unit 630 using an enzymatic method.

The fluidic device 600 in FIG. 6 has an inlet 610 for receiving a solution of a sample. The sample introduced from the inlet 610 is diverted and guided to the Alb sensor unit 620 and the GA sensor unit 630, respectively.

The Alb sensor unit 620 has a first fluid chamber 621. One of the samples diverted from the inlet 610 enters the first fluid chamber 621 and fills it.

The GA sensor unit 630 has two fluid chambers, a second fluid chamber 631 and a third fluid chamber 633, fluidly connected in series. The other of the samples diverted from the inlet 610 first enters the second fluid chamber 631 and fills it, exits the second fluid chamber 631, and then enters the third fluid chamber 633 and fills it.

The first fluid chamber 621 accommodates an albumin measurement device 622 for measuring albumin provided in the present disclosure therein. The albumin measurement device 622 has a recognition unit in which a conductive polymer and BCP or BCG are mixed. As an example, the albumin measurement device 622 may be a current measurement device. As an example, the albumin measurement device 622 may be a biotransistor. An electrode is disposed in the vicinity of the recognition unit. The electrode may be disposed inside the first fluid chamber 621. An analyzer may be disposed outside the first fluid chamber 621.

The second fluid chamber 631 accommodates a protease 632 therein. The third fluid chamber 633 accommodates an enzyme electrode 634 including a ketoamine oxidase and a hydrogen peroxide electrode therein.

The fluidic device 600 in FIG. 6 performs the measurement of albumin and the measurement of glycated albumin for the same sample at different locations. Therefore, for example, measurement conditions such as reaction time, temperature, and time profile thereof can be optimized for each measurement and set independently.

In FIG. 6, the fluidic device 600 has an outlet 641 for discharging the fluid that has exited from the first fluid chamber 621, and an outlet 642 for discharging the fluid that has exited from the third fluid chamber 633. One outlet may be disposed, and the fluid exited from either chamber may be discharged from the outlet. A tank for storing the discharged solution may be provided inside the fluidic device 600 without providing an outlet (not shown).

### Embodiment 2-2: Single Channel

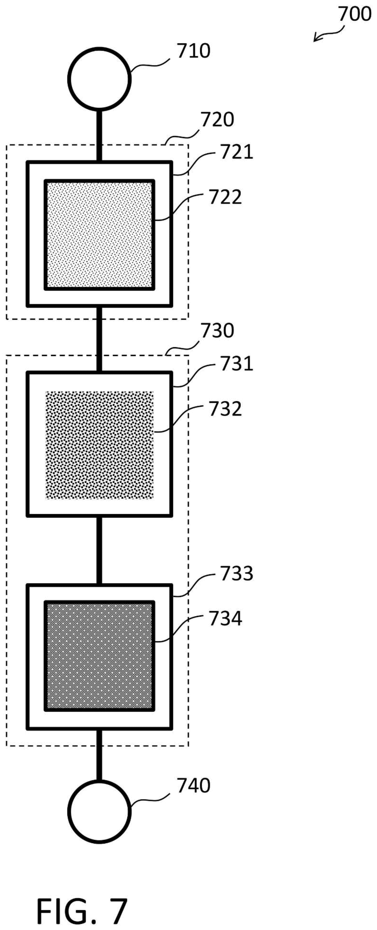

FIG. 7 schematically shows a configuration 700 of a fluidic device for determining a GA level according to an embodiment. The fluidic device 700 includes an albumin (Alb) sensor unit 720 using a conductive polymer and a glycated albumin (GA) sensor unit 730 using an enzymatic method.

In the fluidic device 700 of FIG. 7, an inlet 710, the Alb sensor unit 720, and the GA sensor unit 730 are fluidly connected in series in this order.

The Alb sensor unit 720 has a first fluid chamber 721. A sample introduced from the inlet 710 first enters the first fluid chamber 721 and fills it.

The GA sensor unit 730 has two fluid chambers, a second fluid chamber 731 and a third fluid chamber 732, fluidly connected in series. The sample that has exited from the first fluid chamber 721 enters the second fluid chamber 731 and fills it, exits the second fluid chamber 731, and then enters the third fluid chamber 733 and fills it.

The first fluid chamber 721 accommodates an albumin measurement device 722 for measuring albumin provided in the present disclosure therein. The albumin measurement device 722 has a recognition unit in which a conductive polymer and BCP or BCG are mixed. As an example, the albumin measurement device 722 may be a current measurement device. As an example, the albumin measurement device 722 may be a biotransistor. An electrode is disposed in the vicinity of the recognition unit. The electrode may be disposed inside the first fluid chamber 721. An analyzer may be disposed outside the first fluid chamber 721.

The second fluid chamber 731 accommodates a protease 732 therein. The third fluid chamber 733 accommodates an enzyme electrode 734 including a ketoamine oxidase and a hydrogen peroxide electrode therein.

The fluidic device 700 in FIG. 7 performs the measurement of albumin and the measurement of glycated albumin at different locations but in one channel series. Therefore, for example, measurement conditions such as reaction time, temperature, and time profile thereof can be optimized for each measurement and set independently, and further, the channel system can be designed compactly.

A substance accommodated in the first fluid chamber 721 may exit from the first fluid chamber 721 with the flow of the sample and reach downstream. In that case, it is important that the substance such as BCP used for the measurement of albumin, which was accommodated in the first fluid chamber 721, does not affect the reaction or measurement in the second fluid chamber 731 and/or the third fluid chamber 733 located downstream thereof. Alternatively, the substance accommodated in the first fluid chamber 721 may be disposed or immobilized in the first fluid chamber 721 so as not to exit from the first fluid chamber 721. The inventors have confirmed that BCP and BCG do not affect the degradation of albumin by protease and the reaction between the peptide fragments after degradation and the enzyme electrode in measurement.

In FIG. 7, the fluidic device 700 has an outlet 740 for discharging the fluid that has exited from the third fluid chamber 733. In other embodiments, a tank for storing the discharged solution may be provided inside the fluidic device 700 without providing an outlet (not shown).

### Embodiment 2-3: Two Sensors Disposed in Single Fluid Chamber

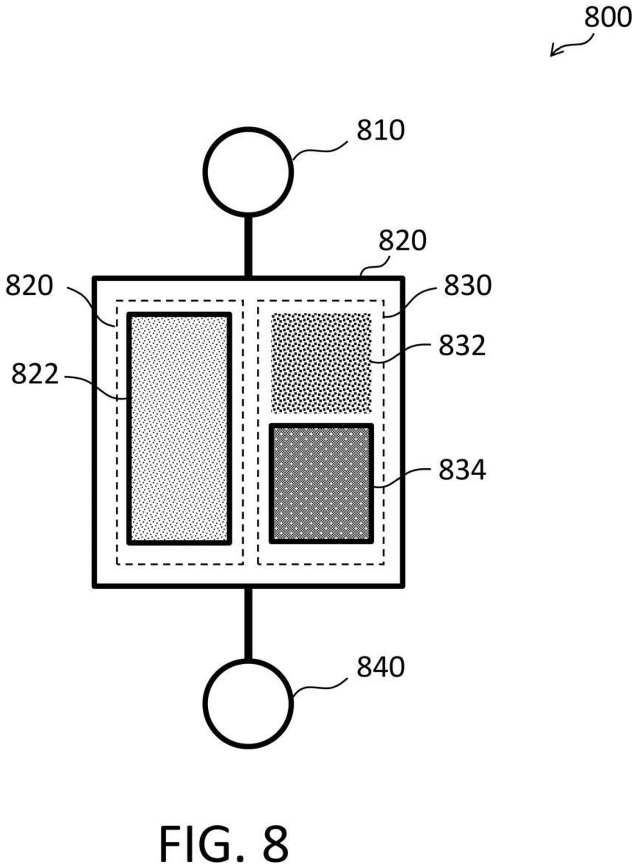

FIG. 8 schematically shows a configuration 800 of a fluidic device for determining a GA level according to an embodiment. The fluidic device 800 in FIG. 8 has a single fluid chamber 815, in which both an albumin (Alb) sensor unit 820 using a conductive polymer and a glycated albumin (GA) sensor unit 830 using an enzymatic method are accommodated.

A sample introduced from an inlet 810 enters the single fluid chamber 815 and fills it.

An albumin measurement device 822 disposed inside the single fluid chamber 815 has a recognition unit in which a conductive polymer and BCP or BCG are mixed. As an example, the albumin measurement device 822 may be a current measurement device. As an example, the albumin measurement device 822 may be a biotransistor. An electrode is disposed in the vicinity of the recognition unit. The electrode may be disposed inside the single fluid chamber 815.

An analyzer may be disposed outside the single fluid chamber 815.

The GA sensor unit 830 disposed inside the single fluid chamber 815 has a protease 832, and an enzyme electrode 834 including a ketoamine oxidase and a hydrogen peroxide electrode. In FIG. 8, the protease 832 and the enzyme electrode 834 are disposed at different locations on a plane. In other embodiments, the protease 832 and the enzyme electrode 834 may be disposed in layers. The arrangement of the GA sensor unit 830, the protease 832, the enzyme electrode 834, and the like inside the single fluid chamber 815 should not be construed as being limited to FIG. 8, and other modes are possible.

There may be a case where an appropriate temperature for the operation of the albumin measurement device 822 is different from appropriate conditions for the protease 832 and the enzyme electrode 834. In such a case, for example, the temperature of the single fluid chamber 815 may be changed to separate the operation of the albumin measurement device 822 and the operations of the protease 832 and the enzyme electrode 834 in time. For example, the albumin measurement device 822 may be first operated at a relatively low temperature (first temperature, T1) to measure the total amount of albumin in the sample, then the reaction of the protease 832 may be performed at a relatively high temperature (second temperature, T2 > T1), and subsequently, the enzyme electrode 834 may be operated at a lower temperature (third temperature, T3 < T2). Thus, a plurality of reactions or device operations may be performed spatially in the single fluid chamber or may be performed at different times.

The fluidic device 800 in FIG. 8 performs the measurement of albumin and the measurement of glycated albumin in one channel chamber. Therefore, the channel system can be designed extremely compactly.

In FIG. 8, the fluidic device 800 has an outlet 840 for discharging the fluid that has exited from the single fluid chamber 815. In other embodiments, a tank for storing the discharged solution may be provided inside the fluidic device 800 without providing an outlet (not shown).

The configurations of the fluidic devices described above are examples, and the invention of the present application should not be construed as being limited thereto. The present disclosure also provides other aspects of the fluidic device.

In some embodiments, the glycated albumin measurement unit or device may be a device comprising the conductive polymer and the dopant of the present disclosure. For example, the conductive polymer is PEDOT:PSS, and the dopant may be a polymer containing a phenylboronic acid group. The phenylboronic acid group recognizes glucose of glycated albumin. Thereby, the electrical property of the conductive polymer changes. By measuring the electrical property, the amount of glycated albumin can be measured.

### Liquid Delivery Mechanism / Liquid Feeder

The device of the present disclosure may have a liquid delivery mechanism or a liquid feeder that moves the introduced solution to an appropriate fluid chamber. Alternatively, the device of the present disclosure may be configured to move the introduced sample to an appropriate fluid chamber by an external liquid delivery mechanism.

In some embodiments, the liquid feeder may be a pusher that pushes a fluid chamber configured to be deformable from the outside. The liquid feeder may compress the liquid chamber, reduce its volume, and feed the liquid accommodated in the fluid chamber to the outside thereof. In some embodiments, the liquid feeder may be a pump. It may be fluidly connected to, or configured to be fluidly connected to, the fluid chamber. In some embodiments, it may be a rotor unit that rotates the fluidic device. The liquid feeder may apply a centrifugal force to the fluidic device to move the liquid inside the fluidic device.

The solution may be moved by positive pressure or negative pressure, or a combination thereof. The positive pressure may be applied to the solution on an upstream side of a predetermined position of the channel. The positive pressure may be applied to the solution on a downstream side of a predetermined position of the channel. The positive pressure and the negative pressure may be applied by a pump. The positive pressure may be applied so as to compress a predetermined position in the channel from the outside. The pressure may be applied by a centrifugal force. For example, the fluidic device may be configured to be mounted on a rotating disk or a unit to be rotated. A centrifugal force is generated by rotation, and the solution in the channel is moved in a direction of the centrifugal force.

FIGS. 6 to 8 schematically show the fluidic devices 600, 700, and 800, and dimensions, sizes, and layouts should not be strictly interpreted in the drawings. In the figures, channels connected to the fluidic device (solid lines in the figures) are drawn linearly and drawn bent at right angles at bending portions, but this should also be understood as schematic. The channel may have a curve. One channel may be composed of a curve, or may be composed of a straight line and a curve. One channel may have a plurality of sub-channels connected at an angle.

The fluidic device may have other chambers. The fluidic device may further have elements having other functions. Such elements may be disposed inside the channel or the chamber, or may be fluidly connected to the channel or the chamber.

In some embodiments, the fluidic device may have a filter or a separation part that removes contaminants from the introduced sample. For example, when the sample is whole blood and albumin in the blood is measured, a filter (e.g., a serum filter or a plasma filter) that removes blood cell components may be disposed upstream of the Alb sensor unit. For example, the filter that removes blood cell components may be disposed upstream of the Alb sensor unit and upstream of the GA sensor unit. For example, when the sample is saliva and albumin in the saliva is measured, a filter that removes contaminants such as mucin and food debris may be disposed upstream of the Alb sensor unit. For example, the filter that removes contaminants such as mucin and food debris may be disposed upstream of the Alb sensor unit and upstream of the GA sensor unit.

The present disclosure includes contents disclosed or described in FIG. A1 to FIG. A23.

The present disclosure includes contents disclosed or described in FIG. B1 to FIG. B3.

The present disclosure includes the following embodiments:
A001. A method for measuring a biomolecule, the method comprising:
   providing a recognition unit comprising a conductive polymer and a molecular recognition pH indicator that acts as a dopant for the conductive polymer and
   recognizes a target biomolecule;
      introducing a sample containing the target biomolecule to the recognition unit;
   measuring an electrical property of the conductive polymer based on a reaction between the target biomolecule and the molecular recognition pH indicator that occurs when the target biomolecule is introduced; and
   determining an amount of the target biomolecule in the sample based on the measured electrical property.
A002. The method according to A001 or any one of the embodiments,
   wherein the molecular recognition pH indicator is disposed within a network of the conductive polymer.
A011. The method according to A001 or any one of the embodiments,
   wherein the recognition unit further comprises a second polymer disposed within a network of the conductive polymer.
A012. The method according to A011 or any one of the embodiments,
   wherein the second polymer has an ability to act as a dopant for the conductive polymer.
A021. The method according to A001 or any one of the embodiments,
   wherein the electrical property is a CV characteristic associated with the reaction between the target biomolecule and the molecular recognition pH indicator.
A022. The method according to A001 or any one of the embodiments,
   wherein the electrical property is a conductive property of the conductive polymer. A101a. The method according to A001 or any one of the embodiments,
   wherein:
      the target biomolecule is albumin; and
      the molecular recognition pH indicator is BCP or BCG.
A101b. A method for measuring albumin, the method comprising:
   providing a recognition unit comprising a conductive polymer and BCP or BCG;
   introducing a sample containing albumin to the recognition unit;
   measuring an electrical property of the conductive polymer based on a reaction between the albumin and the BCP or BCG that occurs when the albumin is introduced; and
   determining an amount of the albumin in the sample based on the measured electrical property.
A105. The method according to A101a or A101b, or any one of the embodiments, wherein the conductive polymer comprises PEDOT.
A106. The method according to A105 or any one of the embodiments,
   wherein the recognition unit further comprises PSS disposed within a network of the conductive polymer.
A201. A method for determining a GA level, the method comprising:
   providing a sample containing albumin;
   determining an amount of albumin in the sample using the method according to any one of A101a to A106 or any one of the embodiments;
   determining an amount of glycated albumin in the sample; and
   determining a GA level of the sample from the amount of albumin and the amount of glycated albumin.
A211. The method according to A201 or any one of the embodiments, wherein determining the amount of glycated albumin comprises:
   introducing a protease to the sample to cause the protease to degrade albumin and generate peptide fragments;
   introducing a ketoamine oxidase to the peptide fragments to generate hydrogen peroxide;
   measuring an amount of the hydrogen peroxide; and
   determining the amount of glycated albumin in the sample from the measured amount of hydrogen peroxide.
B001. A device for measuring a biomolecule, the device comprising:
   a recognition unit comprising a conductive polymer and a molecular recognition pH indicator that acts as a dopant for the conductive polymer and recognizes a target biomolecule; and
   a transducer configured to convert a reaction between the molecular recognition pH indicator and the target biomolecule, which occurs when the target biomolecule is introduced, into an electrical signal.
B005. The device according to B001 or any one of the embodiments, further comprising a circuit connected to the transducer and configured to process the electrical signal based on the reaction between the molecular recognition pH indicator and the target biomolecule.
B011. The device according to B001 or any one of the embodiments,
   wherein:
   the transducer comprises an electrode;
   the conductive polymer and the molecular recognition pH indicator are disposed on a surface of the electrode; and
   the device further comprises a circuit connected to the electrode and configured to evaluate an oxidation-reduction reaction of the conductive polymer associated with the reaction between the molecular recognition pH indicator and the target biomolecule that occurs when the target biomolecule is introduced.
B021. The device according to B001 or any one of the embodiments,
   wherein:
   the device comprises a drain electrode and a source electrode;
   the conductive polymer is disposed to connect the drain electrode and the source electrode; and
   the device comprises a circuit connected to the drain electrode and the source electrode and configured to measure a transistor characteristic between the drain electrode and the source electrode associated with the reaction between the molecular recognition pH indicator and the target biomolecule that occurs when the target biomolecule is introduced.
B101. A device (unit) for measuring albumin, the device (unit) comprising:
   a recognition unit comprising PEDOT and BCP or BCG; and
   a transducer configured to detect a reaction between the BCP or BCG and albumin that occurs when albumin is introduced.
C001. A GA level measurement device comprising:
   the unit for measuring albumin according to B101 or any one of the embodiments; and
   a unit for measuring glycated albumin.
C101. A fluidic device for measuring a GA level, the fluidic device comprising:
   an albumin measurement chamber in which the unit for measuring albumin according to B101 or any one of the embodiments is disposed; and
   a glycated albumin measurement chamber in which a unit for measuring glycated albumin is disposed.
C111. The fluidic device according to C101 or any one of the embodiments, further comprising
   a liquid delivery mechanism configured to introduce a target sample into the albumin measurement chamber and the glycated albumin measurement chamber. C121. The fluidic device according to C101 or C111, or any one of the embodiments,
   wherein:
      the albumin measurement chamber and the glycated albumin measurement chamber are fluidly connected; and
      the glycated albumin measurement chamber is disposed downstream of the albumin measurement chamber.
C131. The fluidic device according to C101 or C111, or any one of the embodiments, configured such that a sample introduced into the fluidic device is diverted and introduced into each of the albumin measurement chamber and the glycated albumin measurement chamber.
C141. The fluidic device according to C101 or C111, or any one of the embodiments,
   further comprising a measurement fluid chamber,
   wherein the measurement fluid chamber accommodates both a unit for measuring albumin and a unit for measuring glycated albumin therein.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Those skilled in the art will occur to numerous variations, changes, and substitutions without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. Therefore, it is contemplated that the invention covers such alternatives, modifications, variations, and equivalents. It is intended that the following claims define the scope of the invention and that methods and structures within the scope of these claims and their equivalents be covered thereby.

## Claims

1. A method for measuring a biomolecule, the method comprising:
providing a recognition unit comprising a conductive polymer and a molecular recognition pH indicator that acts as a dopant for the conductive polymer and recognizes a target biomolecule;
introducing a sample containing the target biomolecule to the recognition unit; measuring an electrical property of the conductive polymer based on a reaction between the target biomolecule and the molecular recognition pH indicator that occurs when the target biomolecule is introduced; and
determining an amount of the target biomolecule in the sample based on the measured electrical property.

2. The method according to claim 1,
wherein the molecular recognition pH indicator is disposed within a network of the conductive polymer.

3. The method according to claim 1,
wherein the recognition unit further comprises a second polymer disposed within a network of the conductive polymer.

4. The method according to claim 3,
wherein the second polymer has an ability to act as a dopant for the conductive polymer.

5. The method according to claim 1,
wherein:
the target biomolecule is albumin; the molecular recognition pH indicator is BCP or BCG; and
the conductive polymer comprises PEDOT.

6. The method according to claim 5,
wherein the recognition unit further comprises PSS disposed within a network of the conductive polymer.

7. A method for determining a GA level, the method comprising:
providing a sample containing albumin;
determining an amount of albumin in the sample using the method according to claim 5 or 6;
determining an amount of glycated albumin in the sample; and
determining a GA level of the sample from the amount of albumin and the amount of glycated albumin.

8. A device for measuring a biomolecule, the device comprising:
a recognition unit comprising a conductive polymer and a molecular recognition pH indicator that acts as a dopant for the conductive polymer and recognizes a target biomolecule; and
a transducer configured to convert a reaction between the molecular recognition pH indicator and the target biomolecule, which occurs when the target biomolecule is introduced, into an electrical signal.

9. The device according to claim 8, further comprising
a circuit connected to the transducer and configured to process the electrical signal based on the reaction between the molecular recognition pH indicator and the target biomolecule.

10. The device according to claim 8,
wherein:
the transducer comprises an electrode;
the conductive polymer and the molecular recognition pH indicator are disposed on a surface of the electrode; and
the device further comprises a circuit connected to the electrode and configured to evaluate an oxidation-reduction reaction of the conductive polymer associated with the reaction between the molecular recognition pH indicator and the target biomolecule that occurs when the target biomolecule is introduced.

11. The device according to claim 8,
wherein:
the device comprises a drain electrode and a source electrode;
the conductive polymer is disposed to connect the drain electrode and the source electrode; and
the device comprises a circuit connected to the drain electrode and the source electrode and configured to measure a transistor characteristic between the drain electrode and the source electrode associated with the reaction between the molecular recognition pH indicator and the target biomolecule that occurs when the target biomolecule is introduced.

12. A device for measuring albumin, the device comprising:
a recognition unit comprising PEDOT and BCP or BCG; and
a transducer configured to detect a reaction between the BCP or BCG and albumin that occurs when albumin is introduced.

13. A device for measuring a GA level, the device comprising:
a unit for measuring albumin, comprising:
a recognition unit comprising PEDOT and BCP or BCG; and
a transducer configured to detect a reaction between the BCP or BCG and albumin that occurs when albumin is introduced; and
a unit for measuring glycated albumin.

14. A fluidic device for measuring a GA level, the fluidic device comprising:
an albumin measurement chamber in which
a recognition unit comprising PEDOT and BCP or BCG, and
a transducer configured to detect a reaction between the BCP or BCG and albumin that occurs when albumin is introduced,
are disposed; and
a glycated albumin measurement chamber in which a unit for measuring glycated albumin is disposed.

15. The fluidic device according to claim 14,
wherein:
the albumin measurement chamber and the glycated albumin measurement chamber are fluidly connected; and
the glycated albumin measurement chamber is disposed downstream of the albumin measurement chamber.

16. The fluidic device according to claim 14,
wherein the fluidic device is configured such that a sample introduced into the fluidic device is diverted and introduced into each of the albumin measurement chamber and the glycated albumin measurement chamber.

17. The fluidic device according to claim 14,
further comprising a measurement fluid chamber,
wherein the measurement fluid chamber accommodates both a unit for measuring albumin and
a unit for measuring glycated albumin therein.
